(19) Europäisches Patentamt / European Patent Office / Office européen des brevets

(11) **EP 4 578 400 A1**

(12) **EUROPEAN PATENT APPLICATION**

(43) Date of publication:
**02.07.2025 Bulletin 2025/27**

(21) Application number: **24223232.0**

(22) Date of filing: **24.12.2024**

(51) International Patent Classification (IPC):
**A61B 6/12** (2006.01)    **A61B 6/46** (2024.01)

(52) Cooperative Patent Classification (CPC):
**A61B 6/12; A61B 6/469**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.12.2023 JP 2023223281**
**20.11.2024 JP 2024202104**

(71) Applicant: **Canon Medical Systems Corporation**
**Tochigi 324-0036 (JP)**

(72) Inventors:
• **ESASHI, Satoru**
  **Otawara-shi, 324-0036 (JP)**
• **IKENO, Toshiyuki**
  **Otawara-shi, 324-0036 (JP)**
• **IIJIMA, Yoshiaki**
  **Otawara-shi, 324-0036 (JP)**
• **MUROI, Toshio**
  **Otawara-shi, 324-0036 (JP)**
• **GUNJI, Masanori**
  **Otawara-shi, 324-0036 (JP)**
• **YAMAJI, Rumiko**
  **Otawara-shi, 324-0036 (JP)**
• **OKADA, Jun**
  **Otawara-shi, 324-0036 (JP)**
• **OGURA, Nobuo**
  **Otawara-shi, 324-0036 (JP)**
• **OHGA, Nobuhiro**
  **Otawara-shi, 324-0036 (JP)**
• **KOIKE, Yuki**
  **Otawara-shi, 324-0036 (JP)**
• **TAKAHASHI, Akihito**
  **Otawara-shi, 324-0036 (JP)**

(74) Representative: **Marks & Clerk LLP**
**15 Fetter Lane**
**London EC4A 1BW (GB)**

(54) **X-RAY DIAGNOSIS APPARATUS AND X-RAY CONDITION SETTING METHOD**

(57)      An X-ray diagnosis apparatus (10) according to an embodiment is provided with: an acquisition unit (110a) configured to acquire a plurality of X-ray images sequentially from a subject (P) with a medical device (A1, A3) inserted into a body of the subject (P); a determination unit (110b) configured to determine a region including the medical device (A1, A3) in the X-ray images; a calculation unit (110c) configured to calculate a degree to which the medical device (A1, A3) is dwelling in the region based on the X-ray images, assign weights to a plurality of pixels included in the region based on the degree, and calculate a parameter related to the region based on the weights and pixel values of the pixels; and a setting unit (110d) configured to set an X-ray condition based on the parameter.

FIG.1

**Description**

FIELD

Embodiments described herein relate generally to an X-ray diagnosis apparatus and an X-ray condition setting method.

BACKGROUND

An X-ray condition setting method called Auto Brightness Control (ABC) is known. The ABC control is a technique that maintains the image quality of X-ray images while reducing the exposure dose to a subject by appropriately adjusting X-ray conditions according to the luminance of previously acquired X-ray images.

In a case where the X-ray conditions are adjusted according to the luminance of the entire X-ray image, the X-ray conditions are not optimal for observing a region of focus because the X-ray conditions are adjusted so that regions other than the region of focus where a user, such as a physician, focuses are also visible. Therefore, in the ABC control, a region of interest (ROI) is usually set on an X-ray image, and X-ray conditions are adjusted according to the luminance in the region of interest. However, there were cases in which regions other than the region of focus were included in the region of interest, and there were also cases in which the region of focus is moved with respect to the region of interest, resulting in inability to set appropriate X-ray conditions.

A problem to be solved by the present invention is to provide an X-ray diagnosis apparatus and an X-ray condition setting method capable of setting an X-ray condition appropriately.

An X-ray diagnosis apparatus according to an embodiment includes an acquisition unit configured to acquire a plurality of X-ray images sequentially from a subject with a medical device inserted into a body of the subject, a determination unit configured to determine a region including the medical device in the X-ray images, a calculation unit configured to calculate a degree to which the medical device is dwelling in the region based on the X-ray images, assign weights to a plurality of pixels included in the region based on the degree, and calculate a parameter related to the region based on the weights and pixel values of the pixels, and a setting unit configured to set an X-ray condition based on the parameter.

Summary of Invention

An X-ray diagnosis apparatus provided according to one aspect of the present invention includes: an acquisition unit configured to acquire a plurality of X-ray images sequentially from a subject with a medical device inserted into a body of the subject; a determination unit configured to determine a region including the medical device in the X-ray images; a calculation unit configured to calculate a degree to which the medical device is dwelling in the region based on the X-ray images, assign weights to a plurality of pixels included in the region based on the degree, and calculate a parameter related to the region based on the weights and pixel values of the pixels; and a setting unit configured to set an X-ray condition based on the parameter.

The calculation unit may determine a movement amount of the medical device between the X-ray images based on a position of the medical device in each of the X-ray images, and evaluate the degree based on the movement amount.

The the calculation unit may determine a length of time when the medical device is dwelling based on a position of the medical device in each of the plurality of X-ray images, and evaluate the degree based on the length of time.

The calculation unit may calculate a parameter related to the region based on a position of a tip end of the medical device in each of the X-ray images and the pixel values.

The calculation unit may calculate a parameter related to the region based on a position of a marker assigned to the medical device in each of the X-ray images and the pixel values.

The calculation unit may assign the weights based on the degree and a time elapsed from determination as the region, and increase the weights of pixels near a tip end of the medical device provided that an amount of change in a size of the region is a threshold value or less for a period of time exceeding the threshold value.

The determination unit may change a shape between a first region and a second region in steps when a dwell mode and a standard mode are switched, wherein: in the dwell mode, the first region including the medical device in the X-ray images is determined as the region, the degree to which the medical device is dwelling in the first region is calculate, the weights is assigned to a plurality of pixels in the first region based on the degree, the parameter is calculated based on the weights and pixel values of the pixels, and the X-ray condition is set based on the parameter; and in the standard mode, a parameter related to the second region, which is predetermined, is calculated based on pixel values of a plurality of pixels included in the second region, and the X-ray condition is set based on the parameter.

The calculation unit may assign a first weight to pixels in a dwell region where the degree is high, assign a second weight smaller than the first weight to pixels in the region except for the dwell region, and replace weights assigned to the pixels in the dwell region by the second weight in a case where a time elapsed from determination as the region exceeds a threshold value.

The determination unit may determine the region by combining a plurality of regions newly determined out of a plurality of regions determined from sequentially acquired X-ray images so that an area of the region does not exceed an upper limit value.

The determination unit may receive an input operation from a user to perform mode switching between a plurality of modes including a dwell mode including assigning the weights to a plurality of pixels included in the region based on the degree.

The determination unit may perform switching to a mode that improves a visibility of a tip end of the medical device provided that an amount of change in a size of the region is a threshold value or less for a period of time exceeding the threshold value.

The determination unit may determine a second region by processing a first region, and the calculation unit may calculate the parameter based on a position of the medical device in each of the X-ray images and pixel values of a plurality of pixels included in the second region.

The X-ray diagnosis apparatus may comprise an output unit configured to display the region on a display unit.

The output unit may sequentially display newly acquired X-ray images on the display unit and display the region on the X-ray images for regular time interval in a superimposing manner.

An X-ray condition setting method provided according to one aspect of the present invention comprises: determining a region including a medical device in a plurality of X-ray images sequentially acquired from a subject with the medical device inserted into a body of the subject; calculating a degree to which the medical device is dwelling in the region based on the X-ray images, assigning weights to a plurality of pixels included in the region based on the degree, and calculating a parameter related to the region based on the weights and pixel values of the pixels; and setting an X-ray condition based on the parameter.

A computer program provided according to one aspect of the present invention causes a computer to execute processes of: determining a region including a medical device in a plurality of X-ray images sequentially acquired from a subject with the medical device inserted into a body of the subject; calculating a degree to which the medical device is dwelling in the region based on the X-ray images, assigning weights to a plurality of pixels included in the region based on the degree, and calculating a parameter related to the region based on the weights and pixel values of the pixels; and setting an X-ray condition based on the parameter.

BRIEF DESCRIPTION OF THE DRAWINGS

FIG. 1 is a block diagram illustrating an example of the configuration of an X-ray diagnosis apparatus according to a first embodiment;
FIG. 2 is a diagram illustrating an example of ROI according to the first embodiment;
FIG. 3 is a diagram illustrating an example of ROI according to the first embodiment;

FIG. 4 is a flowchart illustrating a series of processes executed by processing circuitry according to the first embodiment;
FIG. 5 is a diagram illustrating a region determination process according to the first embodiment;
FIG. 6 is a diagram illustrating the region determination process according to the first embodiment;
FIG. 7 is a diagram illustrating the region determination process according to the first embodiment;
FIG. 8 is a schematic diagram illustrating a trajectory of a tip end of a medical device according to the first embodiment;
FIG. 9 is a diagram illustrating a method for calculating a dwell region according to the first embodiment;
FIG. 10 is a diagram illustrating a method for calculating a dwell region according to the first embodiment;
FIG. 11 is a diagram illustrating an example of a weighting process according to the first embodiment;
FIG. 12 is a diagram illustrating an example of displaying ROI according to the first embodiment;
FIG. 13 is a diagram illustrating an example of displaying the ROI according to the first embodiment;
FIG. 14 is a diagram illustrating an example of UI according to the first embodiment;
FIG. 15 is a diagram illustrating an example of switching ROI according to the first embodiment;
FIG. 16 is a diagram illustrating ROI processing according to a second embodiment;
FIG. 17 is a diagram illustrating the ROI processing according to the second embodiment;
FIG. 18 is a diagram illustrating the ROI processing according to the second embodiment;
FIG. 19 is a diagram illustrating an example of a dwell mode according to a third embodiment;
FIG. 20 is a diagram illustrating an example of a dwell mode according to the third embodiment;
FIG. 21 is a diagram illustrating an example of a dwell mode according to the third embodiment;
FIG. 22 is a diagram illustrating an example of switching between modes according to the third embodiment; and
FIG. 23 is a diagram illustrating an example of ROI according to a fourth embodiment.

DETAILED DESCRIPTION

Hereinbelow, embodiments of an X-ray diagnosis apparatus and an X-ray condition setting method will be described with reference to the drawings. In the following embodiments, parts with the same reference numeral are assumed to operate in the same way, and redundant explanations will not be repeated as appropriate.

First Embodiment

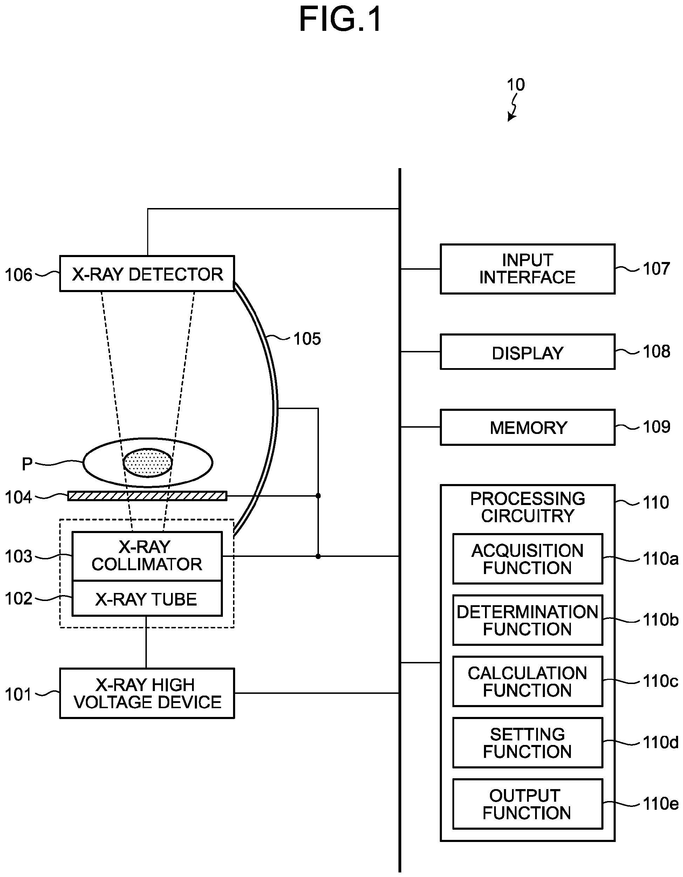

In a first embodiment, an X-ray diagnosis apparatus 10 illustrated in FIG. 1 will be described as an example. FIG. 1 is a block diagram illustrating an example of the configuration of the X-ray diagnosis apparatus 10 according to the first embodiment. As illustrated in FIG. 1, the X-ray diagnosis apparatus 10 is provided with an X-ray high voltage device 101, an X-ray tube 102, an X-ray collimator 103, a couchtop 104, a C-arm 105, an X-ray detector 106, an input interface 107, a display 108, a memory 109, and processing circuitry 110.

The X-ray high voltage device 101 supplies high voltage to the X-ray tube 102 under a control of the processing circuitry 110. For example, the X-ray high voltage device 101 includes a high voltage generating device that includes electrical circuitry such as a transformer and rectifier and generates high voltage to be applied to the X-ray tube 102, and an X-ray control device that controls an output voltage according to X-rays emitted from the X-ray tube 102. The high voltage generating device may be a transformer system or an inverter system.

The X-ray tube 102 is a vacuum tube having a cathode (filament) that generates thermal electrons and an anode (target) that generates X-rays upon collisions of the thermal electrons. The X-ray tube 102 emits thermal electrons from the cathode toward the anode to generate X-rays by using high voltage supplied from the X-ray high voltage device 101.

The X-ray collimator 103 has a collimator that narrows down an irradiation range of X-rays generated by the X-ray tube 102 and a filter that modulates the exposure of X-rays from the X-ray tube 102.

The collimator in the X-ray collimator 103 has, for example, four slidable collimator blades. The collimator slides the collimator blades to narrow down the X-rays generated by the X-ray tube 102 to allow a subject P to be irradiated. Here, the collimator blades are plate-shaped members made of lead or the like and are provided near an X-ray exit port of the X-ray tube 102 to adjust an X-ray irradiation range.

The filter in the X-ray collimator 103 is designed to reduce an exposure dose to the subject P and improve the image quality of X-ray images by changing the beam quality of the X-rays transmitted through the filter depending on a material and a thickness thereof, reducing a soft-ray component that is easily absorbed by the subject P, or reducing a high-energy component that causes contrast reduction in the X-ray images. The filter also changes the dose and irradiation range of the X-rays by a material, a thickness, and a position thereof to attenuate the X-rays so that the X-rays emitted from the X-ray tube 102 to the subject P have a predetermined distribution.

For example, the X-ray collimator 103 has a drive mechanism such as a motor and an actuator, and controls X-ray emission by operating the drive mechanism under a control of the processing circuitry 110 described later. For example, the X-ray collimator 103 controls the irradiation range of X-rays emitted to the subject P by applying a drive voltage to the drive mechanism in response to a control signal received from the processing circuitry 110 to adjust an opening degree of the collimator blades of the collimator. In addition, for example, the X-ray collimator 103 controls the dose distribution of X-rays emitted to the subject P by applying a drive voltage to the drive mechanism in response to a control signal received from the processing circuitry 110 to adjust a position of the filter.

The couchtop 104 is a bed on which the subject P is placed, and is disposed above a couch (not illustrated). The subject P is not included in the X-ray diagnosis apparatus 10. For example, the couch has a drive mechanism such as a motor and an actuator, and controls movement and tilt of the couchtop 104 by operating the drive mechanism under a control of the processing circuitry 110 described later. For example, the couch moves or tilts the couchtop 104 by applying a drive voltage to the drive mechanism in response to a control signal received from the processing circuitry 110.

The C-arm 105 holds the X-ray tube 102 and the X-ray collimator 103, and the X-ray detector 106 opposite to each other with the subject P interposed therebetween. For example, the C-arm 105 has a drive mechanism such as a motor and an actuator, and rotates or moves by operating the drive mechanism under a control of the processing circuitry 110 described later. For example, the C-arm 105 rotates and moves the X-ray tube 102 and the X-ray collimator 103, and the X-ray detector 106 with respect to the subject P by applying a drive voltage to the drive mechanism in response to a control signal received from the processing circuitry 110 to control X-ray irradiation positions and angles. In FIG. 1, the case where the X-ray diagnosis apparatus 10 is, as an example, a single-plane X-ray diagnosis apparatus is exemplified, but the embodiments are not limited to this case, and the X-ray diagnosis apparatus 10 may be a biplane X-ray diagnosis apparatus.

The X-ray detector 106 is, for example, an X-ray flat panel detector (FPD) with detector elements arranged in a matrix. The X-ray detector 106 detects the X-rays emitted from the X-ray tube 102 and transmitted through the subject P, and outputs a detection signal corresponding to the detected X-ray dose to the processing circuitry 110. The X-ray detector 106 may include an indirect-conversion detector with a grid, a scintillator array, and an optical sensor array, or a direct-conversion detector with semiconductor elements that convert incident X-rays into electrical signals.

The input interface 107 receives various input operations from the user, converts the received input operations into electrical signals, and outputs the electrical signals to the processing circuitry 110. For example, the input interface 107 is implemented by a mouse, a keyboard, a trackball, a switch, a button, a joystick, a touch pad for input operation by a touch of an operation surface, a touch screen in which a display screen and a touch pad are integrated, non-contact input circuitry using an optical sensor, voice input circuitry, or the like.

The input interface 107 may be configured as a tablet terminal or the like capable of wireless communication with the processing circuitry 110. The input interface 107 can also be circuitry that receives input operations from the user through motion capture. In one example, the input interface 107 can receive the user's body motion, eye gaze, and the like as input operations by processing signals acquired by a tracker and images acquired about the user. In addition, the input interface 107 is not limited only to those with physical operating components such as a mouse and a keyboard. For example, electrical signal processing circuitry that receives electrical signals corresponding to input operations input from an external input device provided separately from the main body of the X-ray diagnosis apparatus 10 and outputs these electrical signals to the main body of the X-ray diagnosis apparatus 10 is also an example of the input interface 107.

The display 108 displays various types of information. For example, the display 108 displays X-ray images acquired from the subject P. For example, the display 108 also displays a graphical user interface (GUI) for receiving various instructions, settings, and other information from the user via the input interface 107. For example, the display 108 includes a liquid crystal display and a cathode ray tube (CRT) display. The display 108 may include a desktop display or may be configured as a tablet terminal or the like capable of wireless communication with the main body of the X-ray diagnosis apparatus 10.

In FIG. 1, the X-ray diagnosis apparatus 10 is described as being provided with the display 108, but the X-ray diagnosis apparatus 10 may be provided with a projector instead of or in addition to the display 108. The projector can perform projection onto a screen, a wall, a floor, or a body surface of the subject P under a control of the processing circuitry 110. In one example, the projector can also perform projection onto any plane, object, space, or the like by projection mapping. The display 108 or the projector is an example of a display unit.

The memory 109 stores therein various data under a control of the processing circuitry 110. For example, the memory 109 stores therein X-ray images acquired from the subject P. For example, the memory 109 also stores therein computer programs that cause the circuitry included in the X-ray diagnosis apparatus 10 to perform functions thereof. For example, the memory 109 is implemented by, for example, a semiconductor memory device such as a random-access memory (RAM) or a flash memory, a hard disk, an optical disc, or the like. Alternatively, the memory 109 may be implemented by a group of servers (cloud) connected to the X-ray diagnosis apparatus 10 via a network.

The processing circuitry 110 controls all of operations of the X-ray diagnosis apparatus 10 by executing an acquisition function 110a, a determination function 110b, a calculation function 110c, a setting function 110d, and an output function 110e. The acquisition function 110a is an example of an acquisition unit. The determination function 110b is an example of a determination unit. The calculation function 110c is an example of a calculation unit. The setting function 110d is an example of a setting unit. The output function 110e is an example of an output unit.

For example, the processing circuitry 110 reads a computer program corresponding to the acquisition function 110a from the memory 109 and executes the computer program to perform imaging of the subject P and acquire X-ray images. For example, the acquisition function 110a controls the X-ray high voltage device 101 and adjusts a voltage supplied to the X-ray tube 102 to control the dose of X-rays emitted to the subject P, and the on/off status. For example, the acquisition function 110a also controls the irradiation range of X-rays, the dose distribution of X-rays, the irradiation angle of X-rays, and the like by controlling the operations of the X-ray collimator 103, the couchtop 104, the C-arm 105, and the like. Specifically, the acquisition function 110a controls the operation of the X-ray collimator 103 and adjusts the opening degree of the collimator blades included in the collimator, thereby controlling the irradiation range of X-rays with which the subject P is irradiated. The acquisition function 110a also controls the operation of the X-ray collimator 103 and adjusts the position of the filter, thereby controlling the dose distribution of X-rays. The acquisition function 110a also controls the irradiation range and irradiation angle of X-rays by causing the C-arm 105 to rotate or move. The acquisition function 110a also controls the irradiation range and irradiation angle of X-rays by causing the couchtop 104 to move or tilt. The acquisition function 110a also generates X-ray images based on detection signals received from the X-ray detector 106.

Here, the acquisition function 110a can acquire a plurality of X-ray images sequentially. In other words, the acquisition function 110a can acquire the X-ray images over time. For example, the acquisition function 110a can acquire the X-ray images over time by causing the X-ray tube 102 to repeatedly emit pulsed X-rays. For example, the acquisition function 110a acquires the X-ray images from the subject P over time during a procedure in which a medical device is inserted into the body of the subject P (for example, endovascular interventional treatment).

The determination function 110b determines regions in the X-ray images including the medical device. The calculation function 110c calculates a parameter related to the region based on a position of the medical device and pixel values of a plurality of pixels in the region in each of the X-ray images. The setting function 110d sets X-ray conditions based on the parameter. The output function 110e displays various pieces of information, such as the acquired X-ray images and the set X-ray conditions, on the display unit. The details of processes executed by the determination function 110b, the calculation function 110c, the setting function 110d, and the output function 110e are described later.

In the X-ray diagnosis apparatus 10 illustrated in FIG. 1, each of the processing functions is stored in the memory 109 in the form of a computer program executable by a computer. The processing circuitry 110 is a processor that reads each computer program from the memory 109 and executes the computer program to implement a function corresponding to each computer program. In other words, the processing circuitry 110 with a computer program read out has a function corresponding to the read computer program.

In FIG. 1, the processing circuitry 110 is described as the single processing circuit that implements the acquisition function 110a, the determination function 110b, the calculation function 110c, the setting function 110d, and the output function 110e; however, the processing circuitry 110 may be configured by combining a plurality of independent processors, and each processor may execute a computer program to implement a function. In addition, the individual processing functions of the processing circuitry 110 may be implemented in a distributed manner or integrated into a single processing circuit or a plurality of processing circuits, as appropriate.

The processing circuitry 110 may also use a processor of an external device connected via the network to perform a function. For example, the processing circuitry 110 reads out each computer program corresponding to each function from the memory 109 and executes the computer program, and uses a group of servers connected to the X-ray diagnosis apparatus 10 via the network as a computing resource to implement each function illustrated in FIG. 1.

The configuration example of the X-ray diagnosis apparatus 10 has been described above. Under such a configuration, the X-ray diagnosis apparatus 10 enables appropriate setting of the X-ray conditions.

It is to be understood that a process of "increasing" and "being large" in the following description may be relative. For example, the process of "increasing" a weight assigned to a pixel may be a process of changing the weight assigned to the pixel to a larger value, a process of changing weights assigned to other pixels to a smaller value, or a combination of these processes. The same applies to expressions such as "reducing" and "being small".

In the related art, automatic brightness control (ABC) has been known as a technique for setting X-ray conditions appropriately. For example, in the ABC control, a region of interest (ROI) is set on an X-ray image, and X-ray conditions are set based on pixel values in the ROI.

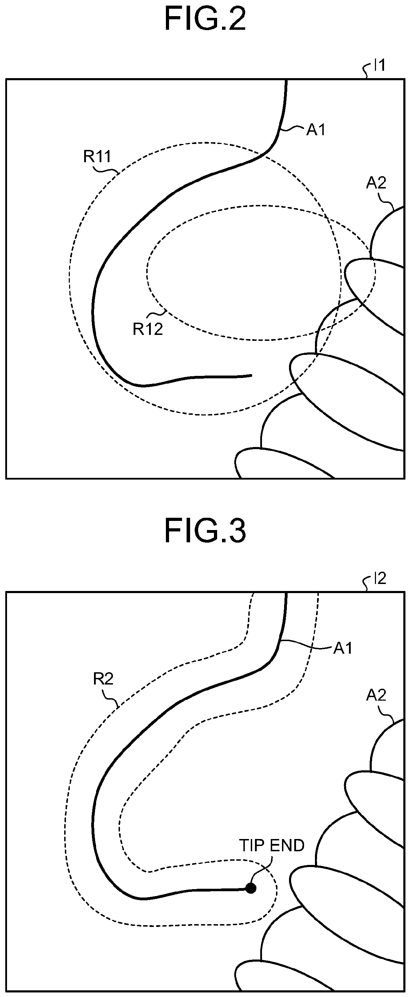

An example of the ABC control is illustrated in FIG. 2. An X-ray image I1 illustrated in FIG. 2 is an X-ray image acquired from the subject P with a medical device A1 inserted in the body of the subject P. The medical device A1 is, for example, a catheter, guidewire, or the like inserted into a blood vessel of the subject P when endovascular interventional treatment is carried out. In FIG. 2, a circular region R11 is set in the center of the X-ray image I1 as an ROI. The region R11 is, for example, a default ROI where a position and a shape are preset. For the region R11 illustrated in FIG. 2, a certain weight is assigned to each pixel in the region R11.

For example, in the ABC control, a parameter such as the average pixel value is calculated for pixels included in the region R11, and the X-ray conditions are set based on the comparison result of the calculated parameter with a predetermined threshold value. The pixel value used to calculate the parameter in the ABC control may be a value (luminance) corresponding to the X-ray dose detected by the X-ray detector 106, or may be a value after any conversion process is performed on the luminance. That is, the pixel value is a value assigned to each pixel based on the X-ray detection result from the X-ray detector 106 and is not necessarily the luminance itself.

The X-ray conditions set in the ABC control are, for example, a tube voltage, tube current, emission time, and the like of X-rays emitted from the X-ray tube 102. For example, in a case where the average value of the luminance of the pixels included in the region R11 is lower than the threshold value (that is, in a case where the region R11 is dark), the tube voltage is increased to increase the X-ray transmission rate, or the tube current or the emission time are increased to increase the X-ray dose. Therefore, the X-ray conditions are changed so that in X-ray images acquired after the X-ray image I1, the average value of the luminance of the pixels contained in the region R11 is higher than the threshold value (that is, the region R11 becomes brighter).

However, the region R11 illustrated in FIG. 2 does not necessarily match a region of focus where the user focuses. In many cases, the user operates the medical device A1 with a focus on the current tip end position of the medical device A1 or positions through which the tip end of the medical device A1 has passed to reach the current position. Hereinbelow, the positions through which the tip end of the medical device A1 has passed to reach the current position is also simply described as a trajectory.

In contrast, although the region R11 in FIG. 2 is disposed at an appropriate position, including the tip end of the medical device A1, it is not necessary to focus on, for example, a position included in a region R12 in the operation of the medical device A1. That is, adjusting the X-ray conditions to enable the clear visibility even at the position in the region R12, included in the region R11, is not optimal as the ABC control for observing the region of focus.

In addition, for example, in a case where an interfering object such as an object A2 illustrated in FIG. 2 is included in the region R11, the X-ray conditions are set so that the interfering object is easily visible, resulting in the reduced visibility of the region of focus. This is particularly noticeable in a case where the interfering object is a highly absorbent object such as a bone, a tooth, or a medical device other than the medical device A1. Examples of medical devices other than the medical device A1 include pacemakers, artificial joints, and transesophageal echocardiography (TEE) probes.

As described above, it is preferable that the ROI is set to match the region of focus where the user focuses, as illustrated in the region R2 in FIG. 3, for example. The region R2 is an ROI set based on the trajectory of the tip end of the medical device A1. Specifically, the region R2 is set by the determination function 110b to encompass the trajectory of the tip end of the medical device A1. As illustrated in FIG. 3, the region R2 does not include an interfering object such as the region R12 or the object A2 illustrated in FIG. 2. Therefore, X-ray conditions suitable for viewing the region of focus can be set by executing the ABC control using the region R2 as the ROI.

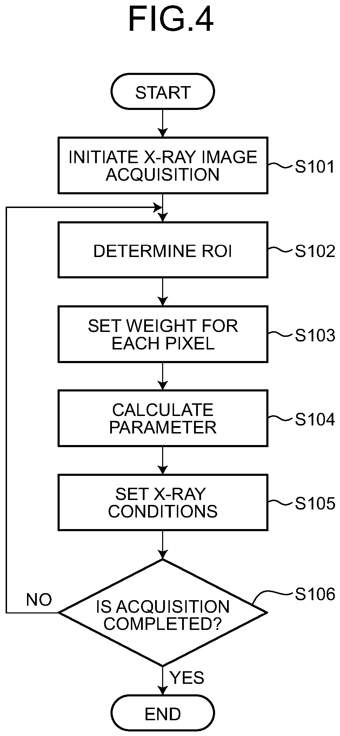

The ABC control performed by the X-ray diagnosis apparatus 10 will be described in more detail below according to the flowchart in FIG. 4. First, X-ray image acquisition is initiated at step S101 as illustrated. After step S101, a plurality of X-ray images are acquired over time while processes at steps S102 to S106 are performed. Alternatively, steps S102 to S106 may be performed every time one or a predetermined number of X-ray images are acquired.

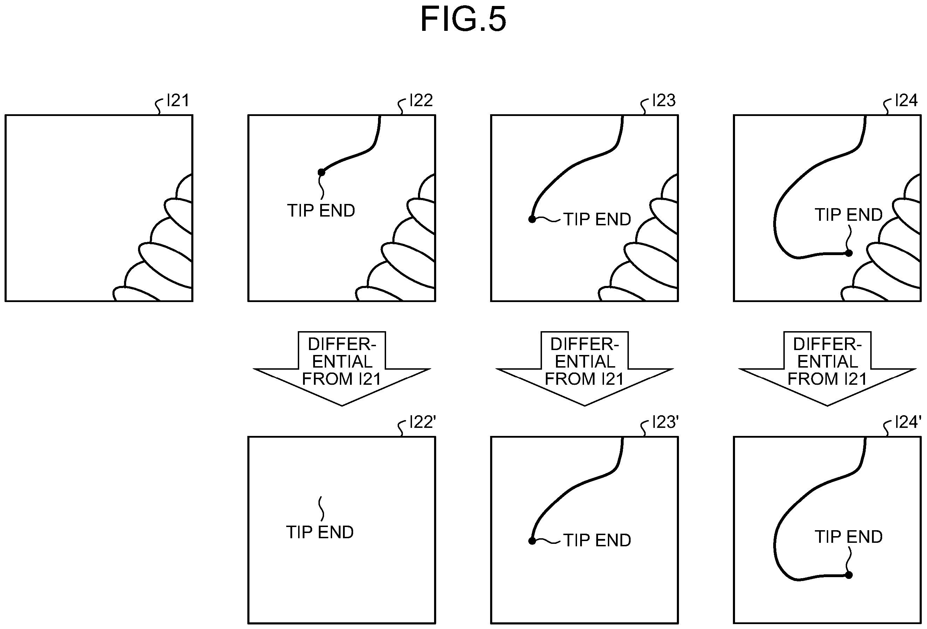

At step S102, the determination function 110b determines a region in the X-ray images including the medical device. That is, at step S102, the determination function 110b sets the ROI to include the medical device in the X-ray images. Hereinbelow, a region determination process by the determination function 110b will be described using FIGS. 5 to 7. In FIGS. 5 to 7, as with the region R2 in FIG. 3, an example of setting an ROI based on the trajectory of the tip end of the medical device A1 will be described.

An X-ray image I21, an X-ray image I22, an X-ray image I23, and an X-ray image I24 illustrated in FIG. 5 are examples of the X-ray images acquired over time. For example, the X-ray images I21 to I24 are images of four consecutive frames or four-frame images extracted at regular sampling intervals from a plurality of frames of the X-ray images.

More specifically, the X-ray image I21 is an image acquired before the medical device A1 reaches the imaging range. Thereafter, the X-ray image I22, the X-ray image I23, and the X-ray image I24 are acquired in sequence while the operation of inserting the medical device A1 is underway. The X-ray images I21 to I24 may be acquired under ABC-controlled X-ray conditions based on the default ROI illustrated in FIG. 2, or may be acquired under default X-ray conditions without the ABC control.

As illustrated in FIG. 5, the determination function 110b differentiates the X-ray image I21, as a mask image, from each of the X-ray image I22, the X-ray image I23, and the X-ray image I24. The differential image between the X-ray image I22 and the X-ray image I21 is also described as an X-ray image I22'. The differential image between the X-ray image I23 and the X-ray image I21 is also described as an X-ray image I23'. The differential image between the X-ray image I24 and the X-ray image I21 is also described as an X-ray image I24'. In the X-ray image I22', the X-ray image I23', and the X-ray image I24', background components such as bones and organs are removed, and the medical device A1 is emphasized.

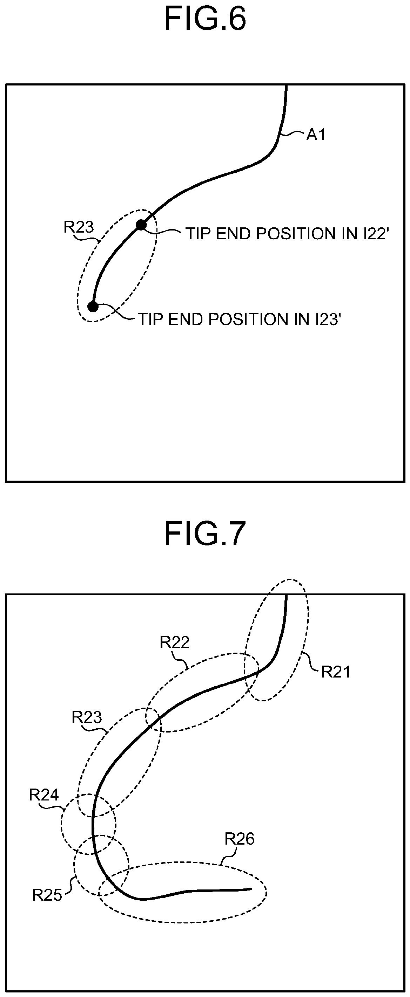

The determination function 110b sets a region including the medical device A1 based on the tip end positions of the medical device A1 in the X-ray image I22', the X-ray image I23', and the X-ray image I24'. For example, the determination function 110b sets a region R23 that includes the tip end position in the X-ray image I22' and the tip end position in the X-ray image I23', as illustrated in FIG. 6. In one example, the determination function 110b sets, as the region R23, an elliptical region including two focal points of the tip end position in the X-ray image I22' and the tip end position in the X-ray image I23'. In another example, the determination function 110b sets, as the region R23, a region included at a certain distance from the line segment connecting the tip end position in the X-ray image I22' and the tip end position in the X-ray image I23'.

As in FIG. 6, the determination function 110b can set a region including the medical device A1 with respect to an X-ray image for each frame. That is, the determination function 110b can sequentially set a plurality of regions as illustrated in the regions R21 to R26 in FIG. 7 based on the tip end position of the medical device A1 in each of a plurality of X-ray images. A region with the regions R21 to R26 combined corresponds to the region R2 illustrated in FIG. 3. That is, the X-ray conditions suitable for viewing the region of focus can be set by executing the ABC control using the region with the regions R21 to R26 combined, as an ROI.

In FIG. 5, the example of using the X-ray image I21 acquired before the medical device A1 reaches the imaging range as a mask image is described, but the mask image can be updated as needed. For example, in a case where the X-ray image I22 is used as the mask image, in the differential images from the X-ray image I23 and the X-ray image I24, the background components such as bones and organs as well as the portion of the medical device A1 that has been depicted in the X-ray image I22 are removed. For example, in the differential image between the X-ray image I22 and the X-ray image I23, only the portion of the medical device A1 ahead of the tip end position at the time of acquisition of the X-ray image I22 is depicted.

The region determination method illustrated in FIGS. 5 to 7 is only an example, and there may be various modifications. For example, in a case where the medical device A1 is a wire-shaped device such as a catheter or a guidewire, the trajectory of the tip end of the medical device A1 generally matches the shape of the medical device A1. Thus, the determination function 110b may determine the ROI based on the shape of the medical device A1 depicted in the X-ray image. For example, the determination function 110b may determine the ROI by performing image processing, such as segmentation processing or image recognition processing, on X-ray images such as the X-ray images I22 to I24 illustrated in FIG. 5, or differential images from the mask image. The image processing may be performed by a trained model (for example, a trained neural network for implementing a semantic segmentation).

In addition, the region determination process may be performed by the determination function 110b on a pixel-by-pixel basis in the X-ray image, or on a unit different from the pixel. For example, the determination function 110b may set a section composed of a plurality of pixels on the X-ray image with the arrangement in a grid pattern and determine the region including the medical device A1 by using the section as a unit.

Although the ROI set based on the trajectory of the tip end of the medical device A1 is the region where the user focuses, the degree of focus is not constant within the ROI. For example, the degree of focus in the region near the tip end of the medical device A1 is higher as compared to the proximal end side of the medical device A1.

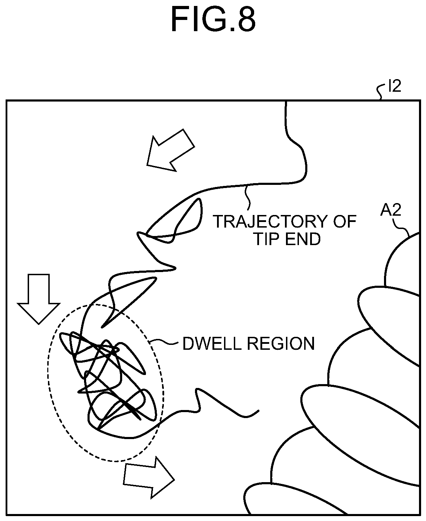

The degree of focus on the dwell region illustrated in FIG. 8 is also higher. FIG. 8 is a schematic diagram illustrating the trajectory of the tip end of the medical device A1 when the operation of inserting the medical device A1 is performed in the directions of the arrows. The dwell region is a region where it is difficult to advance the medical device A1 and a region through which the medical device A1 took time to pass through. The dwell region may continue to be the subject of focusing even after the tip end has passed.

There are various factors that make it difficult to advance the medical device A1 in the dwell region, examples thereof include a case where the dwell region is a characteristic part of the vascular structure (for example, a curved part, a branched part, or the like). Such a characteristic part may continue to remain in focus even after the tip end has been passed through, for example, to avoid stress on a blood vessel wall during the operation of the medical device A1.

In addition, other examples thereof include a case where the dwell region is a region to be treated. For example, when the interventional treatment for a blood vessel at a stenotic site, the tip end of a guide wire is passed through the stenotic site to carry a balloon, a stent, or other device along the guide wire to the stenotic site Here, it is assumed as a case that the medical device A1 is a guidewire, and takes time to pass through a stenotic site.

As described above, from the viewpoint that the degree of focus differs for each position, the calculation function 110c sets a weight for each pixel (step S103). For example, the calculation function 110c sets a larger weight for pixels near the tip end of the medical device A1. In addition, for example, the calculation function 110c sets large weights for pixels in the dwell region. That is, the calculation function 110c calculates the degree to which the medical device A1 was dwelling in the region determined at step S102, and assigns weights to a plurality of pixels in the region based on the degree.

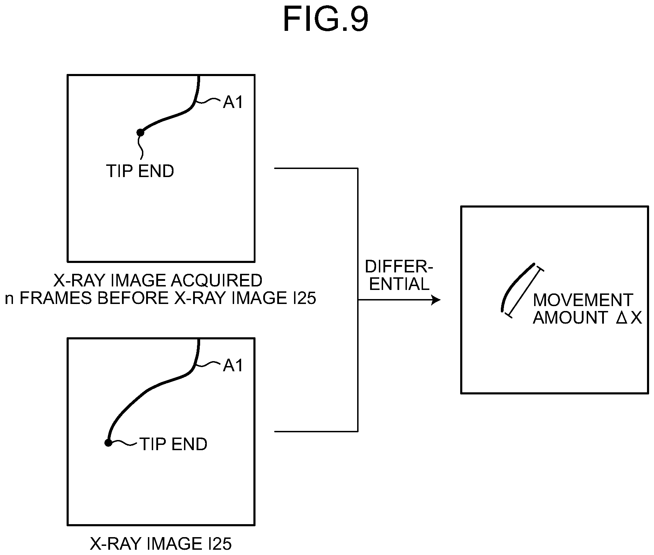

Here, an example of a calculation method for the dwell region will be described with reference to FIG. 9. FIG. 9 illustrates an X-ray image I25 acquired from the subject P and an X-ray image acquired an n frame (n frames) before the X-ray image I25. Here, n is a natural number. That is, the X-ray image acquired an n frame (n frames) before the X-ray image I25 may be an X-ray image from the frame immediately before the X-ray image I25, or an X-ray image from two or more frames before the X-ray image I25. In addition, each X-ray image illustrated in FIG. 9 may be an image that has been subjected to differential processing from the mask image, or may be an image that has not been subjected to the differential processing.

As illustrated in FIG. 9, the differential processing is performed between the X-ray image I25 and the X-ray image acquired n frames before the X-ray image I25 to extract a tip end portion of medical device A1. The length of this tip end portion corresponds to a movement amount ΔX of the medical device A1 between n frames. The calculation function 110c can identify the dwell region based on the movement amount ΔX. For example, in a case where the movement amount ΔX is lower than the threshold value, the calculation function 110c identifies a region around the tip end portion of the medical device A1 extracted by the differential processing as the dwell region.

The process of calculating the dwell region can be rephrased as a process of calculating the degree to which the medical device A1 was dwelling by using two-level ranking of "whether to correspond to the dwell region or not". For example, the region identified as the dwell region is a region where the degree to which the medical device A1 was dwelling is higher than the threshold value, and the region not identified as the dwell region is a region where the degree to which the medical device A1 was dwelling is lower than the threshold value. The degree to which the medical device A1 was dwelling can also be calculated by using a three or more-level ranking, or no-level ranking. As an example, the present embodiment describes the example in which a process of calculating the degree to which the medical device A1 was dwelling is performed by using the two-level ranking (whether to correspond to the dwell region or not).

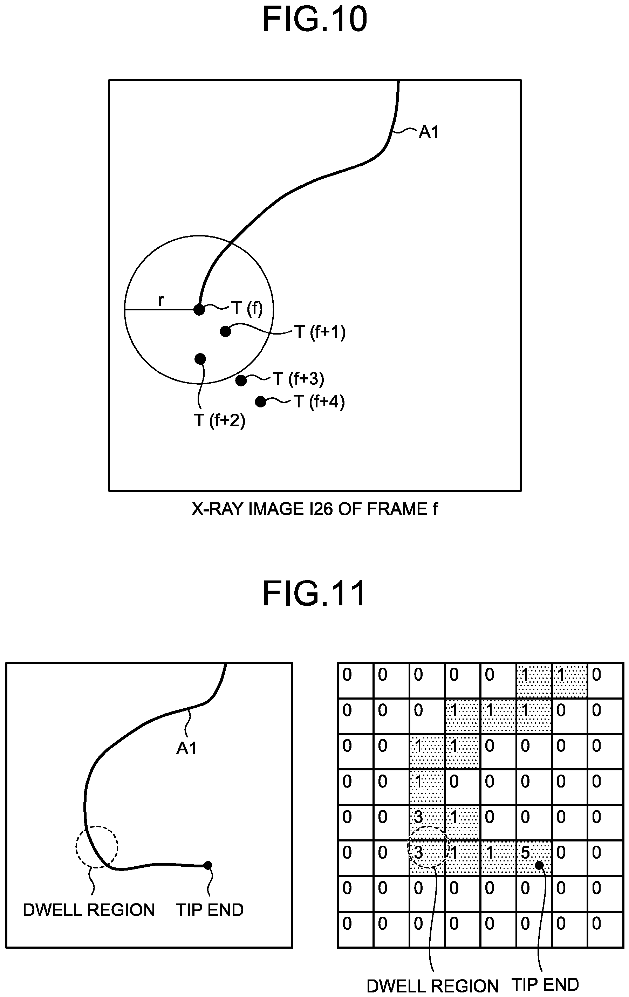

Next, FIG. 10 is used to illustrate another example of the calculation method for the dwell region. FIG. 10 illustrates an X-ray image I26 of a frame f. In addition, in the X-ray image I26, the tip end position of the medical device A1 at the time of the frame f is denoted by "T(f)", the tip end position of the medical device A1 at the time of a frame (f+1) is denoted by "T(f+1)", the tip end position of the medical device A1 at the time of a frame (f+2) is denoted by "T(f+2)", the tip end position of the medical device A1 at the time of a frame (f+3) is denoted by "T(f+3)", and the tip end position of the medical device A1 at the time of a frame (f+4) is denoted by "T(f+4)".

The calculation function 110c also calculates the length of time when the medical device A1 was dwelling by using "r" illustrated in FIG. 10 as the threshold value. That is, the calculation function 110c calculates a dwell time with respect to the position "T(f)" by assuming that the medical device A1 was dwelling while the tip end position is included in a circle of radius "r" centered on the tip end position "T(f)". For example, in the case illustrated in FIG. 10, the medical device A1 is dwelling for a period corresponding to two frames from the frame f to the frame (f+2). The calculation function 110c compares the number of frames in which the medical device A1 was dwelling or the number of seconds in which the number of frames is converted according to the frame rate with the threshold value, and identifies a region around the tip end position "T(f)" as the dwell region in a case where the number of frames or the number of seconds is larger than the threshold value.

The calculation function 110c then assigns weights to the pixels included in the ROI based on the calculation results of the dwell region. In the following description, the ROI in which each of the pixels is given a weight is also described as a weighted ROI.

FIG. 11 is used to illustrate an example of a process of assigning a weight. The left diagram in FIG. 11 illustrates an X-ray image, and the right diagram illustrates the distribution of weights set for the pixels of the X-ray image in the left diagram. The distribution of the weights of the right diagram in FIG. 11 is an example of a weighted ROI. The weights may be assigned per pixel or per section including a plurality of pixels. In FIG. 11, an example in which the X-ray image is divided into 64 sections of "8 × 8" and a weight is assigned to each of the sections.

In FIG. 11, at least a weight of "1" is assigned to the pixels within the ROI, and a weight of "0" is set for the pixels outside the ROI. The ROI in FIG. 11 is a region determined based on the trajectory of the tip end of the medical device A1, for example, as illustrated in FIGS. 5 to 7. As illustrated in FIG. 11, if medical device A1 is a wire-shaped device, the ROI determined based on the trajectory is shaped along medical device A1.

For the pixel corresponding to the "tip end", the calculation function 110c sets a weight of "5" as it is the position of focus for the user. The "tip end" is the current tip end position of the medical device A1.

In addition, for the pixel corresponding to the "dwell region", the calculation function 110c sets a weight of "3" as it is the position of focus for the user. Here, the "dwell region" is a region identified as a dwell region in the current or previous X-ray image. For example, as illustrated in FIG. 11, the medical device A1 has already passed through the dwell region, but the calculation function 110c also sets a weight of "3" for the region previously identified as the dwell region.

The weighting process illustrated in FIG. 11 is only an example, and there may be various modifications.

For example, when the endovascular interventional treatment is carried out, the calculation function 110c may detect an indwelling device from the X-ray image and may set a large weight to the pixel at the position of the indwelling device. For example, when the endovascular interventional treatment is carried out for the purpose of dilating a blood vessel at a stenotic site, a balloon catheter is inserted into the blood vessel of the subject P. The balloon catheter causes the blood vessel to dilate by allowing a balloon to reach the stenotic site and inflating the balloon. The balloon catheter also indwells a stent in the blood vessel at the stenotic site so that the blood vessel remains in a dilated state. In a case where the medical device A1 is the balloon catheter, the calculation function 110c detects the indwelling device such as the balloon or the stent from the X-ray image and sets a large weight to the pixel at the detected position. For example, the calculation function 110c can detect the indwelling device by performing image processing, such as pattern matching, on a marker assigned to the indwelling device or a shape of the indwelling device itself.

The calculation function 110c may also perform an addition so that the weight is increased when the position to which the large weight is set (for example, the tip end of the medical device A1, the dwell region, the indwelling device, or the like) as described above overlaps with a highly absorbent object such as a bone. In contrast, for the position in the ROI where the highly absorbent object do not overlap with the position where the large weight is set, the small weight may be set, for example, by setting a weight of "0".

In addition, the calculation function 110c may set large weights for pixels in a vascular region. There is no particular limitation on the calculation method for the vascular region, but as one example, the calculation is carried out based on the trajectory of the tip end of the medical device A1. For example, in a case where the ROI is set by extending the positions on the trajectory as illustrated in FIGS. 5 to 7, the entire ROI does not necessarily correspond to the vascular region, but it can be said that at least the positions on the trajectory are included in the vascular region since the medical device A1 inserted in the blood vessel was able to be positioned. Therefore, the calculation function 110c sets a near region narrower than the ROI in the vicinity of the trajectory, and sets a larger weight to the near region as the vascular region than to the other positions in the ROI.

Of course, in a case where angiographic images are available, the calculation function 110c can identify the vascular region based on the angiographic images. For example, the acquisition function 110a can inject a contrast agent into the blood vessel of the subject P while acquiring a plurality of X-ray images over time, thereby using the acquired X-ray images as the angiographic images. The injection of the contrast agent may be performed by the acquisition function 110a controlling an injector, or the user may perform the injection manually. In addition, for example, in a case where a 3D contrast vessel model has been acquired in advance, the calculation function 110c can identify the vascular region by aligning the X-ray images acquired over time by the acquisition function 110a with the 3D contrast vessel model.

Although the example of increasing the weight is described as a modification of weighting, the calculation function 110c may reduce the weight as appropriate in consideration of various factors. For example, FIGS. 5 to 7 illustrate an example in which the regions R21 to R26 are set sequentially and combined to form an ROI. Here, the region R26 including the current tip end of the medical device A1 is a new region set based on the real-time images, whereas in the region R21, a period of time has elapsed since the region R21 was set. In this way, in a case where the time elapsed from the setting differs for each position in the ROI, the calculation function 110c may reduce the weight of the position with the long elapsed time.

For example, it is preferable to assign a large weight to the tip end of the medical device A1 while the medical device A1 is being operated, because the tip end is focused on. However, when the operation of the medical device A1 is stopped, the degree of focus is reduced. Thus, for example, in a case where the tip end of the medical device A1 does not move for a certain period of time, the calculation function 110c may reduce the weight assigned to the tip end of the medical device A1.

After assigning the weights to the pixels in the ROI, the calculation function 110c calculates a parameter related to the ROI based on the assigned weights and pixel values (step S104). For example, the calculation function 110c calculates a weighted average value as a parameter related to the ROI by using the following Expression (1). In Expression (1), a pixel with a weight of "0" may be excluded from the calculation using Expression (1).

$$\sum_{i \in ROI} w_i p_i \Big/ \sum_{i \in ROI} w_i \quad \cdots (1)$$

A subscript i in Expression (1) is an argument indicating a pixel. In addition, the ROI in Expression (1) is a region in the X-ray image excluding pixels with a weight of "0" set. A weight of "0" may also be set to the pixels within the ROI as well as a weight of "0" is set to the pixels outside the ROI. A $w_i$ in Expression (1) is a weight (weighting factor) assigned to an i-th pixel. In addition, $p_i$ in Expression (1) is a pixel value of the i-th pixel.

In Expression (1), the example of calculating the average value as a parameter related to the ROI has been described, but specific examples of the parameter are not limited thereto. For example, the calculation function 110c may calculate a statistical value other than the average value as a parameter, or for example, may calculate other values such as a contrast-to-noise ratio (CNR).

In addition, for example, although the example of setting the ROI shaped along the trajectory of the tip end of the medical device A1 has been described as illustrated in FIGS. 3 and 7, the embodiments are not limited thereto. For example, the determination function 110b may set a circular region similar to FIG. 2 as the ROI. Even in such a case, a weight of "0" is set to the pixels at the position spaced from the trajectory of the tip end of the medical device A1 (for example, the pixels in the region R12), out of the circular regions, so that a weighted ROI includes the same content. As a result, the same value as that in a case of using the ROI in FIGS. 3 and 7 as the parameter related to the ROI is calculated.

The setting function 110d sets the X-ray conditions based on the parameter calculated by the calculation function 110c (step S105). For example, the setting function 110d compares the parameter with the threshold value and sets the X-ray conditions based on the comparison result. The threshold value is stored in advance in the memory 109, for example. The threshold value can be set, changed, or adjusted as appropriate by a user's instruction via the input interface 107. For example, the setting function 110d adjusts at least one of the tube voltage or the product of the tube current and the X-ray emission time (tube current-time product mAs) based on the comparison result between the parameter and the threshold value. That is, the setting function 110d performs the ABC control based on the parameter related to the ROI.

In a case where the X-ray conditions are set at step S105, the acquisition function 110a performs the acquisition of a subsequent X-ray image according to the newly set X-ray conditions. In addition, the processing circuitry 110 determines whether or not the acquisition of the X-ray image is completed (step S106), and when the acquisition is completed (affirmative at step S106), the process is terminated. On the other hand, when the acquisition is not completed (negative at step S106), the processing circuitry 110 returns to step S102 again.

In a case where the process returns to step S102 again, the determination function 110b determines the ROI again. For example, in a case where the processes at steps S102 to S106 have been performed using the combined regions R21 to R26 illustrated in FIG. 7 as the ROI, and the process has returned to step S102 again, it is assumed that the position of the tip end of the medical device A1 in the newly acquired X-ray image moves further ahead of the position illustrated in FIG. 7. In this case, the determination function 110b can set a region based on the position of the tip end of the medical device A1 in the newly acquired X-ray image and combine the region with the regions R21 to R26 to produce a new ROI.

The ROI may be reset as appropriate. That is, even though the position is once determined to be included in the ROI, the degree of focus may be reduced over time, and the position may no longer be appropriate to be included in the ROI. For example, in a case where the user receives an instruction to reset the ROI, the determination function 110b determines a new ROI rather than updating the existing ROI. For example, the determination function 110b sets a new ROI based on the trajectory of the tip end of the medical device A1 that appears in an X-ray image composed of a predetermined number of frames including the newly acquired X-ray image.

The output function 110e may display the ROI determined by the determination function 110b as appropriate. For example, the output function 110e sequentially performs display on the display 108 each time a new X-ray image is acquired by the acquisition function 110a. That is, the output function 110e performs real-time display of the X-ray image. Furthermore, the output function 110e superimposes the ROIs on the X-ray image that is being displayed in real time.

For example, the output function 110e displays the ROIs for regular time interval (for example, every minute). By superimposing the ROIs on the X-ray image that is being displayed in real time, it may be easier to recognize what kind of ROI has been set, but may also interfere with the visibility of the X-ray image. By displaying the ROIs for regular time interval, the output function 110e can ensure the visibility of the X-ray image while presenting the ROIs to the user in a way of easy recognition. In addition, for example, in a case where the change in an ROI is small (for example, no more than 10% difference) since the previous display, the output function 110e may omit the display of the ROI. Alternatively, the output function 110e may display the ROI only in a case where a change by the threshold value or more has occurred on the ROI.

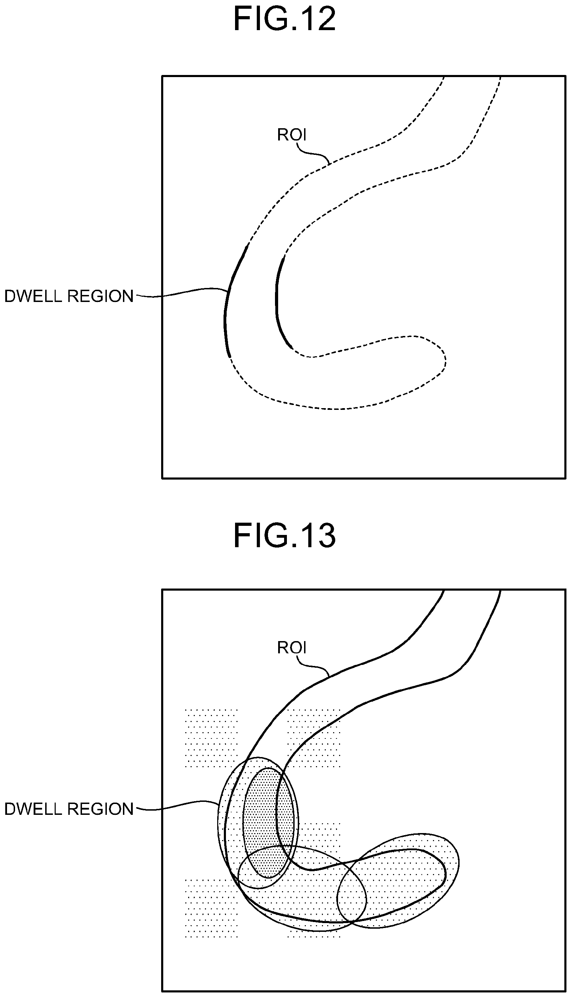

Examples of the ROI display are illustrated in FIGS. 12 and 13. In FIG. 12, an ROI is illustrated as the dashed line and a dwell region is illustrated as the solid lines. According to the display in FIG. 12, the user can understand that a particularly large weight is assigned to the dwell region along with the range of the set ROI. In FIG. 13, an ROI is illustrated as the solid line, and the magnitude of the weight assigned to pixels at each position is illustrated in color. That is, in FIG. 13, the weighted ROI is displayed. According to the display in FIG. 13, the user can understand the range of the set ROI and the distribution of weights assigned to the positions.

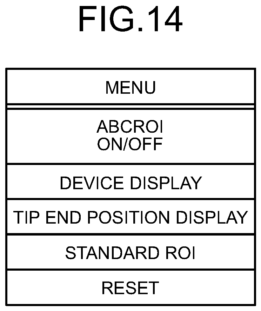

For example, in a case where the user does not focus on the dwell region in FIG. 12 or a case where the distribution of the weights in FIG. 13 does not match with the user's preference, the user can instruct to change the ROI. For example, the output function 110e displays a user interface (UI) illustrated in FIG. 14 on the display 108. The user can give an instruction to change the ROI by manipulating the UI in FIG. 14 via the input interface 107.

For example, in a case where "STANDARD ROI" illustrated in the UI in FIG. 14 is selected, the determination function 110b determines the default ROI illustrated in FIG. 2, for example, as a new ROI. That is, when "STANDARD ROI" is selected, the ABC control is performed based on the predetermined region. In addition, in a case where "RESET" illustrated in the UI in FIG. 14 is selected, the determination function 110b resets the ROI as described above. That is, when "RESET" is selected, the determination function 110b does not update the existing ROI, but rather determines a new ROI.

In addition, in a case where "ABC ROI ON/OFF" illustrated in the UI in FIG. 14 is selected, the output function 110e toggles between showing and hiding the ROI illustrated in FIG. 12 or 13. In a case where "DEVICE DISPLAY" illustrated in the UI in FIG. 14 is selected, the output function 110e highlights the medical device A1. In addition, in a case where "TIP END POSITION DISPLAY" illustrated in the UI in FIG. 14 is selected, the output function 110e highlights the tip end of the medical device A1.

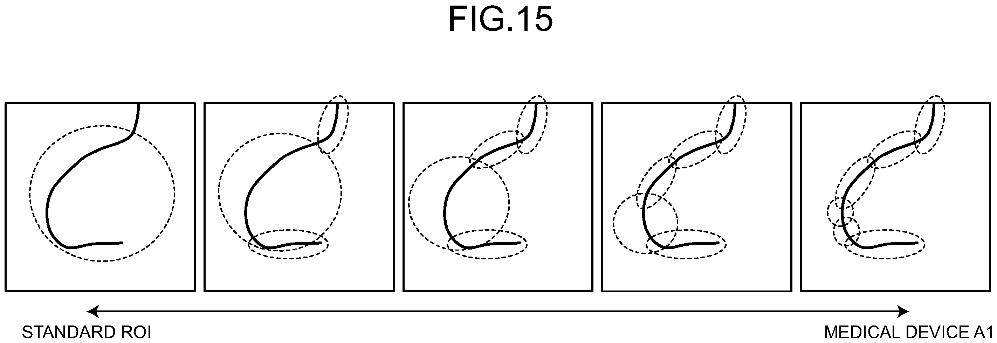

Note that, by switching the ROI, such as the case of selecting "STANDARD ROI" in FIG. 14, a significant change may occur in the X-ray conditions set by the ABC control, resulting in an abrupt change in the appearance (brightness, contrast, or the like) of the displayed image. In order to reduce such an abrupt change in the image appearance, the determination function 110b may switch the ROI in stages, as illustrated in FIG. 15, for example.

As noted above, the processing circuitry 110 can perform the ABC control based on the region containing the medical device A1. For example, the determination function 110b determines the combined regions R21 to R26 illustrated in FIG. 7 as an ROI. In addition, the calculation function 110c calculates the degree to which the medical device A1 was dwelling in the ROI. For example, the calculation function 110c identifies a dwell region in the ROI. Next, the calculation function 110c assigns weights to a plurality of pixels in the ROI based on the degree to which the medical device A1 was dwelling, and calculates a parameter related to the ROI based on the assigned weights and pixel values. The setting function 110d then performs the ABC control based on the parameter related to the ROI. A mode that includes assigning the weights to the pixels in the ROI based on the degree to which the medical device A1 was dwelling in the ROI is also described as a dwell mode.

The processing circuitry 110 can also perform the ABC control based on a predetermined region (STANDARD ROI). For example, the determination function 110b determines the circular region R11 illustrated in FIG. 2 as an ROI. In addition, the calculation function 110c calculates a parameter related to the region R11 based on pixel values of the pixels in the circular region R11. For example, the calculation function 110c calculates a statistical value of the pixel values of the pixels included in the region R11 as a parameter related to the region R11. Although specific examples of the statistical value are not particularly limited, a mean or a median can be exemplified as an example. The setting function 110d then performs the ABC control based on the parameter related to the circular region R11. The mode in which the ABC control is performed using a predetermined region as the ROI is also described as a standard mode.

The region including the medical device A1, which is determined as the ROI in the dwell mode, is also described as a first region. For example, the combined region including the regions R21 to R26 illustrated in FIG. 7 is an example of the first region. In addition, the predetermined region determined as the ROI in the standard mode is also described as a second region. For example, the circular region R11 illustrated in FIG. 2 is an example of the second region.

The dwell mode and the standard mode can be switched to each other as appropriate. For example, in a case where "STANDARD ROI" in FIG. 14 is selected while the ABC control is being performed in the dwell mode, the determination function 110b switches to the ABC control in the standard mode.

Of course, the determination function 110b may also switch from the standard mode to the dwell mode. For example, immediately after the start of X-ray image acquisition or when "RESET" illustrated in FIG. 14 is selected, the determination function 110b first sets the standard mode and switches the standard mode to the dwell mode in steps in accordance with the X-ray image acquisition proceeding. In addition, for example, in a case where "STANDARD ROI" in FIG. 14 is selected while the ABC control is being performed in the standard mode, the processing circuitry 110 may switch the standard mode to the ABC control in the dwell mode. Alternatively, in the UI of FIG. 14, an icon may be further displayed to switch from the standard mode to the dwell mode.

When switching between the dwell mode and the standard mode, the determination function 110b may change the shape between the first and second regions in steps. For example, when switching from the dwell mode to the standard mode, the determination function 110b changes the shape of the ROI in steps so that the shape progressively becomes closer to the standard ROI from the region including the medical device A1, as illustrated in FIG. 15. For example, the determination function 110b changes the shape of the ROI step by step each time one or a predetermined number of new X-ray images is acquired. Similarly, when switching from the standard mode to the dwell mode, the determination function 110b may change the shape of the ROI in steps so that the shape progressively becomes closer to the region including the medical device A1 from the standard ROI.

As described above, the acquisition function 110a of the first embodiment acquires the X-ray images sequentially from the subject P into which the medical device A1 is inserted. In addition, the determination function 110b determines the ROI in the X-ray images including the medical device. In addition, the calculation function 110c calculates, based on the X-ray images, the degree to which the medical device A1 was dwelling in the ROI, assigns the weights to the pixels in the ROI based on the calculated degree, and calculates the parameter related to the ROI based on the assigned weights and pixel values. In addition, the setting function 110d sets the X-ray conditions based on the parameters related to the ROI. With such a configuration, the X-ray diagnosis apparatus 10 according to the first embodiment enables appropriate setting of the X-ray conditions.

For example, the calculation function 110c identifies the dwell region based on the position of the tip end of the medical device A1 in each of the X-ray images and sets the weighted ROI to assign the large weight to the pixels included in the dwell region. The X-ray diagnosis apparatus 10 can perform the ABC control to improve the visibility of the dwell region according to such a weighted ROI. Furthermore, by improving the visibility of the region of focus such as the dwell region, the medical device A1 can be operated smoothly. As a result, the procedure time can be shortened, and the exposure dose to the subject P can be reduced.

Second Embodiment

In the first embodiment, the example of determining the region including the medical device A1 as the ROI has been described. In the second embodiment, a second region is determined by processing a first region, and the second region after the processing is used as an ROI to perform various processes such as parameter calculation. Hereinafter, the same reference numerals will be assigned to the parts with the same descriptions as those of the first embodiment, and the descriptions thereof will not be repeated.

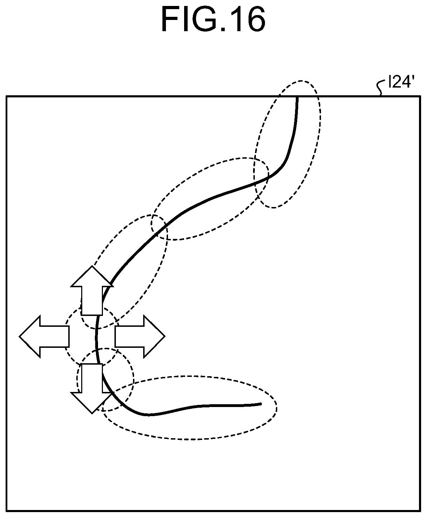

In one example, the determination function 110b performs the above-described processing based on a dwell region. For example, the determination function 110b first sets a plurality of regions illustrated by the dotted lines in FIG. 16 in the same manner as in FIGS. 5 to 7. The regions illustrated by the dotted lines in FIG. 16 are examples of the first region. In addition, the calculation function 110c identifies the dwell region based on the position of the tip end of a medical device A1 in each of a plurality of X-ray images.

Here, the determination function 110b extends a region out of the regions illustrated by the dotted lines in FIG. 16, which overlaps with the dwell region, and determines the region after the extension as an ROI. Such an extended ROI is an example of the second region. Therefore, the ABC control can be executed so that the visibility is improved, including a region around the dwell region. In a case where a highly absorbent object such as bone is positioned around the dwell region, the determination function 110b may not perform the extension to avoid the highly absorbent object being included in the ROI to affect the ABC control.

In another example, the determination function 110b performs the above-described processing based on a change in the relative position or angle between the X-ray tube 102 or the X-ray detector 106 and a subject P. For example, in a case where the couchtop 104 undergoes translation (panning), the subject P also undergoes translation, and the position relative to the X-ray tube 102 and the X-ray detector 106 changes. In this case, the determination function 110b can perform processing on an ROI based on the X-ray images before and after the couchtop 104 undergoes translation, and determine the processed ROI as a new ROI. For example, the determination function 110b aligns background components such as bone between the X-ray images before and after the couchtop 104 undergoes translation, calculates the amount of shift, and shifts the ROI accordingly.

The X-ray detector 106 may also be able to move and rotate on a plane parallel to a detection plane. In this case, the determination function 110b can calculate the amount of ROI shift using general image processing calculation (affine transform, or the like) and can process the ROI because the amount of movement and the amount of rotation of the X-ray detector 106 are known values controlled by the acquisition function 110a.

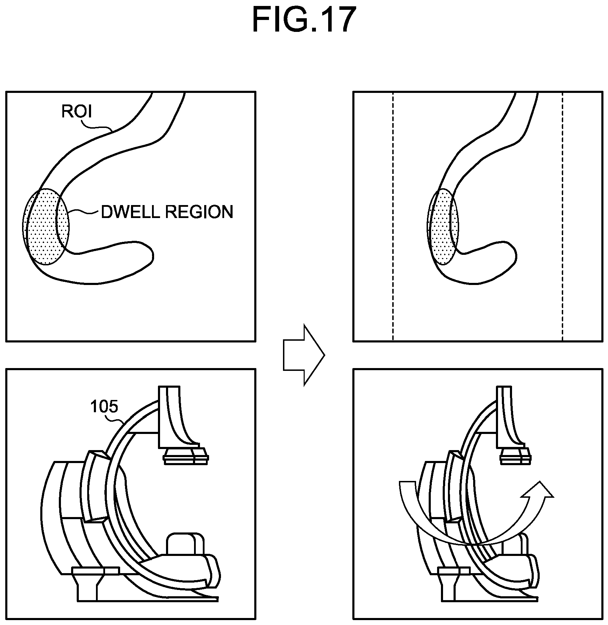

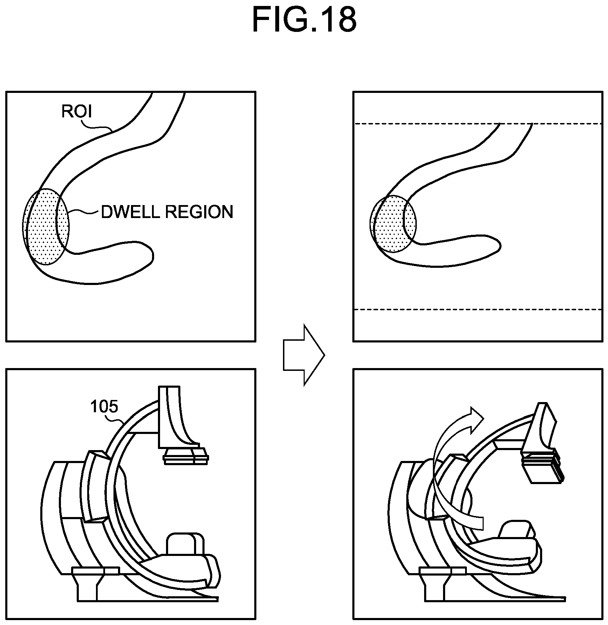

In addition, the movement of the C-arm 105 causes the X-ray tube 102 and the X-ray detector 106 to rotate and move with respect to the subject P. The determination function 110b pseudo-follows three-dimensional changes in the ROI based on the direction and amount of rotation of the C-arm 105, for example, as illustrated in FIGS. 17 and 18, and changes the shape and position of the ROI. The calculation method for the three-dimensional changes in the ROI is not particularly limited, and can be implemented by general image transformation processing. It is assumed that there is a case where it is not possible to follow the three-dimensional changes in the ROI, such as a case where the amount of rotation of the C-arm 105 is large or a case where a region of focus including the medical device A1 or the like is out of the X-ray irradiation range due to the rotation of the C-arm 105. In this case, the determination function 110b can set a predetermined second region (for example, the default ROI illustrated in FIG. 2). In other words, in a case where it is difficult to continue the ABC control in the dwell mode, the determination function 110b can perform switching to the standard mode.

Third Embodiment

In FIG. 15, the example of switching between the dwell mode and the standard mode has been described. However, the embodiments are not limited thereto, and switching between modes is possible for various other modes. A third embodiment describes various modes that can be performed on the X-ray diagnosis apparatus 10 and a switching method between the modes. Hereinafter, the same reference numerals will be assigned to the parts with the same descriptions as those in the first and second embodiments, and the descriptions thereof will not be repeated.

An example of a mode that can be implemented in addition to the dwell mode and the standard mode includes a tip end priority mode. In the tip end priority mode, the determination function 110b determines an ROI to include the tip end of a medical device A1. In addition, the calculation function 110c assigns the largest weights to the vicinity of the tip end of the medical device A1 or assigns weights only to the vicinity of the tip end of the medical device A1, and calculates a parameter related to the ROI based on the assigned weights and pixel values. The setting function 110d then performs the ABC control based on the parameter related to the ROI.

The ABC control is executed in the tip end priority mode to improve the visibility of the tip end position of the medical device A1. However, in the tip end priority mode, the visibility of a dwell region is reduced because the degree to which the medical device A1 was dwelling is not taken into account. For example, the determination function 110b can receive, from a user, an instruction to switch between the tip end priority mode and the dwell mode, or the switching between the tip end priority mode and the standard mode, via a UI similar to that in FIG. 14. Alternatively, in a case where it is difficult to continue the ABC control in the dwell mode by the C-arm 105 rotating, the determination function 110b may automatically perform switching to the tip end priority mode.

In addition, so far, the example of switching between the dwell mode, the standard mode, and the tip end priority mode has been described. However, these modes can be further subdivided into a plurality of modes. For example, a plurality of types of dwell modes may be provided, and the determination function 110b may perform mode switching among these dwell modes.

For example, as described in the first embodiment, the calculation function 110c can assign weights to a plurality of pixels in the ROI to reduce the weights of positions that have a longer elapsed time from the determination as the ROI. That is, the calculation function 110c can perform a dwell mode with consideration of the time elapsed. In contrast, the calculation function 110c can also assign weights to the pixels in the ROI based on the degree to which the medical device A1 was dwelling in the ROI, without consideration of the time elapsed from the determination as the ROI. Therefore, the determination function 110b may perform the switching between the dwell mode with consideration of the time elapsed and the dwell mode without consideration of the time elapsed, based on an input operation received from the user, for example.

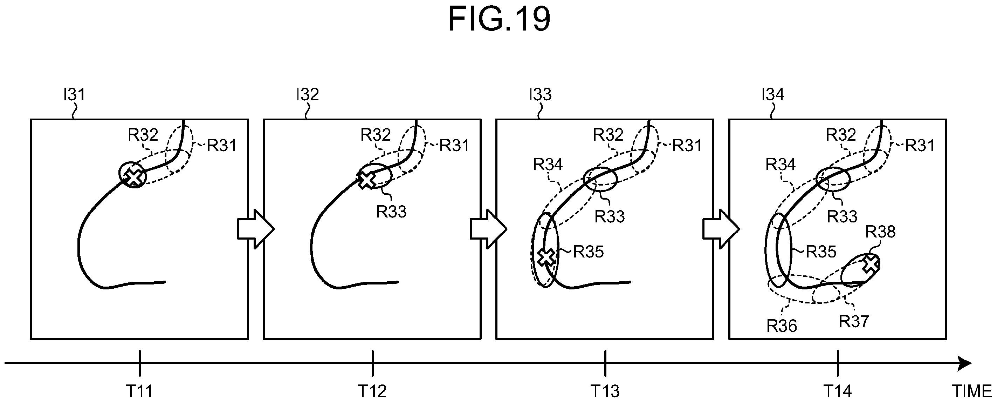

The dwell mode with consideration of the time elapsed will be described with reference to FIG. 19. FIG. 19 is a diagram illustrating an example of the dwell mode according to the third embodiment. In FIG. 19, an X-ray image I31, an X-ray image I32, an X-ray image I33, and an X-ray image I34 sequentially acquired are illustrated. In addition, the tip end position of a medical device A1 is illustrated with a cross mark in each of the X-ray images I31 to I34.

In FIG. 19, a region R31 and a region R32 are determined as ROIs at a time T11. The regions R31 and R32 are determined based on a plurality of X-ray images, including the X-ray image I31. For example, the region R31 is determined based on one or more X-ray images at a time prior to the time T11. In addition, at the time T11, the region R32 is determined based on the X-ray image I31 or the X-ray images including the X-ray image I31.

In addition, the ROI determined at the time T11 is a combined region including the region R31 and the region R32. In FIG. 19, the region R31 and the region R32 are overlapped. The overlapped region may be included in the recently determined region R32 or in the previously determined region R31. In a case where one of the two regions overlapped with each other is a dwell region, the overlapped region may be included in the dwell region.

In addition, in FIG. 19, a region R33 is added as an ROI at a time T12. The region R33 may be determined based only on the X-ray image I32 or on the X-ray images including the X-ray image I32. The ROI determined at the time T12 is a combined region including the region R31, the region R32, and the region R33.

Similarly, in FIG. 19, a region R34 and a region R35 are added as ROIs at a time T13. In addition, a region R36, a region R37, and a region R38 are added as ROIs at a time T14. At the time T14, a combined region including the regions R31 to R38 is the ROI. In addition, as a result of the calculation of the degree to which the medical device A1 was dwelling, the region R33, region R35, and region R38 are identified as dwell regions.

For example, the calculation function 110c first assigns a weight to each pixel based on the degree to which the medical device A1 was dwelling. The weighting process at the time T14 in FIG. 19 is described as an example. The calculation function 110c performs the weighting in the same manner as in the case illustrated in FIG. 11, for example. That is, the calculation function 110c assigns a weight of "3" to the pixels in the region R33, region R35, and region R38 serving as the dwell regions, as these pixels are positions where the user focuses. Alternatively, a weight of "5" may be assigned to the pixels in the region R38 because the region R38 contains the tip end of medical device A1. In addition, the calculation function 110c assigns a weight of "1" to the pixels in the ROIs except for the region R33, the region R35, and the region R38. Note that a weight of "0" is assigned to the pixels outside the ROI.

Furthermore, the calculation function 110c corrects the weight assigned to each of the pixels based on the degree to which the medical device A1 was dwelling, based on the time elapsed from the determination as an ROI. In FIG. 19, it is assumed that the region R31 is first determined as an ROI, followed by the region R32, the region R33, the region R34, the region R35, the region R36, the region R37, and the region R38 sequentially. That is, at the time T14, the region R38 is the newest region with the shortest time elapsed from the determination as an ROI. In this case, the calculation function 110c corrects the weight assigned to the pixel in each region so that the weight of the pixel in the region R38 is increased.

Hereinbelow, the weight assigned to each pixel based on the degree to which the medical device A1 was dwelling will be described as a pre-correction weight. The pre-correction weight is assigned in the same manner as in the case illustrated in FIG. 11, for example. In addition, the weight corrected based on the time elapsed from the determination of each region will be described as a post-correction weight.

For example, the calculation function 110c calculates the post-correction weight by multiplying the pre-correction weight by a coefficient based on the time elapsed. For example, the calculation function 110c assigns a coefficient of "1" to the region R38 with the most recent and shortest elapsed time at the time T14. The calculation function 110c also assigns coefficients to regions other than the region R38 so that the longer the elapsed time, the coefficients are smaller than "1" and closer to "0". For example, a coefficient close to "1" is assigned to the region R37 that has been determined immediately before the determination of the region R38. In addition, for the first determined region R31, a coefficient close to "0" is assigned. Therefore, the post-correction weight is calculated to be increased as the degree of dwelling is higher, and increased as the elapsed time is shorter.

The calculation function 110c then calculates the parameter related to the ROI based on the post-correction weight assigned to each pixel and the pixel value of each pixel. In addition, the setting function 110d performs the ABC control based on the parameter related to the ROI.

In the dwell mode illustrated in FIG. 19, the ABC control is performed to ensure a well-balanced visibility between the dwell region and the current or most recent position of the medical device A1. For example, in the case illustrated in FIG. 19, the ABC is executed with the highest priority given to the visibility of the region R38 that includes the current position of the medical device A1 and is also a dwell region. In addition, in a case where the region R38 was not a dwell region, the ABC control is performed so that both the region R38 that includes the current position of the medical device A1, and the other dwell regions (region R33 and region R35) can be well-balanced for visibility.

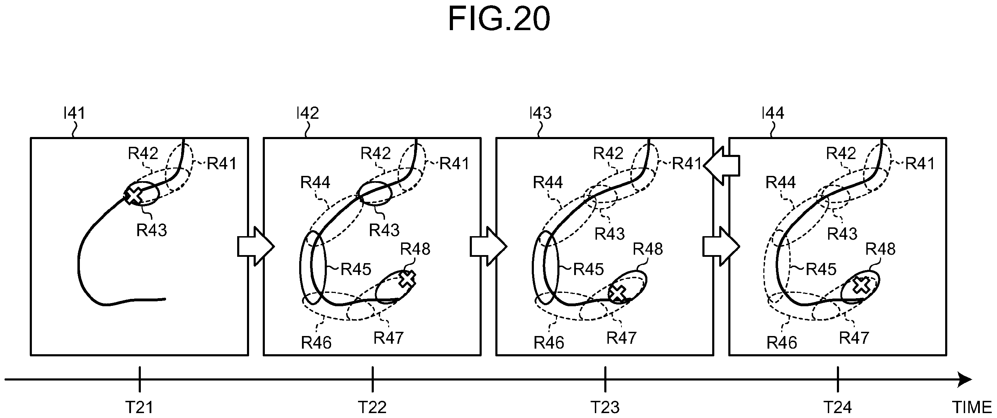

Another example of the dwell mode with consideration of the time elapsed will be described with reference to FIG. 20. FIG. 20 is a diagram illustrating an example of a dwell mode according to the third embodiment. In FIG. 20, X-ray images I41, I42, I43, and I44 sequentially acquired are illustrated. In addition, the tip end position of a medical device A1 is illustrated with a cross mark in each of the X-ray images I41 to I44.

In FIG. 20, a region R41, a region R42, and a region R43 are determined as ROIs at a time T21. As in FIG. 19, the region R41, the region R42, and the region R43 are determined based on a plurality of X-ray images, including the X-ray image I41. For example, at a time prior to the time T21, the region R41 is determined based on one or more X-ray images, the region R42 is determined based on the next one or more X-ray images acquired, and at the time T21, the region R43 is determined based on the X-ray image I41 or the X-ray images including the X-ray image I41. The ROI determined at the time T21 is a combined region including the region R41, the region R42, and the region R43. In addition, in FIG. 20, a region R44, a region R45, a region R46, a region R47, and a region R48 are added to ROIs at a time T22.

The ROI determined at the time T22 is a combined region including the regions R41 to R48. In addition, as a result of the calculation of the degree to which the medical device A1 was dwelling, the region R43, region R45, and region R48 are identified as dwell regions. The calculation function 110c assigns weights to the pixels included in the ROIs, for example, as in the case illustrated in FIG. 11. That is, the calculation function 110c assigns a weight of "3" to the pixels in the region R43, region R45, and region R48 serving as the dwell regions, as these pixels are positions where the user focuses. Alternatively, a weight of "5" may be assigned to the pixels in the region R48 because the region R48 contains the tip end of medical device A1. In addition, the calculation function 110c assigns a weight of "1" to the pixels in the ROIs except for the region R43, the region R45, and the region R48. Note that a weight of "0" is assigned to the pixels outside the ROI.

In FIG. 20, the calculation function 110c reduces the weight assigned to the earlier dwell regions sequentially. For example, at each of times such as a time T23 and a time T24, the calculation function 110c compares the elapsed time from the determination of each of the dwell regions such as the region R43, the region R45, and the region R48 as an ROI with the threshold value. The calculation function 110c then reduces the weight assigned to the dwell region where the elapsed time exceeds the threshold value.

Among the dwell regions illustrated in FIG. 20, the region R43 is determined first as the ROI, followed by the region R45, and finally the region R48. Therefore, at the time T23, in only the region R43, the time equal to or more than the threshold value has elapsed from the determination as an ROI. Therefore, the calculation function 110c assigns the same weight to the pixels in the region R43 after the time T23 as to the pixels in the ROIs except for the dwell regions. For example, in a case of assigning a weight of "3" to the pixels in the region R43 at the time T22, the calculation function 110c assigns a weight of "1" to the pixels in the region R43 after the time T23. A weight of "3" is an example of a first weight assigned to the pixels in the dwell regions. A weight of "1" is an example of a second weight assigned to the pixels in the ROI except for the dwell regions. That is, the calculation function 110c assigns the first weight to the pixels in the dwell regions where the degree to which the medical device A1 was dwelling is high, assigns the second weight to the pixels in the ROI except for the dwell regions, and in a case where the time elapsed from the determination as an ROI exceeds the threshold value, the weights assigned to the pixels in the dwell regions are replaced by the second weights.

Similarly, at the time T24, a time equal to or more than the threshold value has elapsed from the determination of the region R45 as an ROI. Therefore, the calculation function 110c assigns the same weights to the pixels in the region R45 after the time T24 as to the pixels in the ROIs except for the dwell regions. That is, the calculation function 110c resets the weighting as a dwell region in the order from the earliest one when a certain time has elapsed.

In the dwell mode illustrated in FIG. 20, the ABC control is basically performed to improve the visibility of a dwell region. However, for the earlier dwell regions whose time elapsed from the determination as the ROIs exceeds the threshold value, the priority is reduced as the degree of focus has been decreased. Here, when it is assumed that multiple dwell regions are identified depending on the situation of the procedure, there is a probability that the visibility of other regions of focus, such as the tip end of the medical device A1, is reduced in a case where the large weights are assigned to all of these multiple dwell regions. In contrast, since the dwell mode illustrated in FIG. 20 reduces the weight assigned to the earlier dwell region whose elapsed time exceeds the threshold value, the visibility of the other regions of focus can be ensured.

Note that the calculation function 110c may retain the information that the region R43 is a dwell region after the time T23. Similarly, the calculation function 110c may retain the information that the region R45 is a dwell region after the time T24. Then, for example, the control may be switched to weight the regions R43 and R45 as the dwell regions according to an input operation from the user appropriately.

For example, in FIG. 20, at the time T24, a large weight is assigned to only the region R48 as the dwell region. For example, a weight of "3" is assigned to the pixels in the region R48, and a weight of "1" is assigned to the pixels in the ROIs other than the region R48. That is, at the time T24, the ABC control is performed with the highest priority on the visibility of the region R48 that is the only dwell region whose time elapsed from the determination as the ROI does not exceed the threshold value. Here, in a case where the user intends to check the other dwell regions as well, the calculation function 110c can increase the weight assigned to the pixels in the region R45 as in the case of the image I43 in FIG. 20, or increase the weights assigned to the pixels in the region R43 and region R45 as in the image I42.

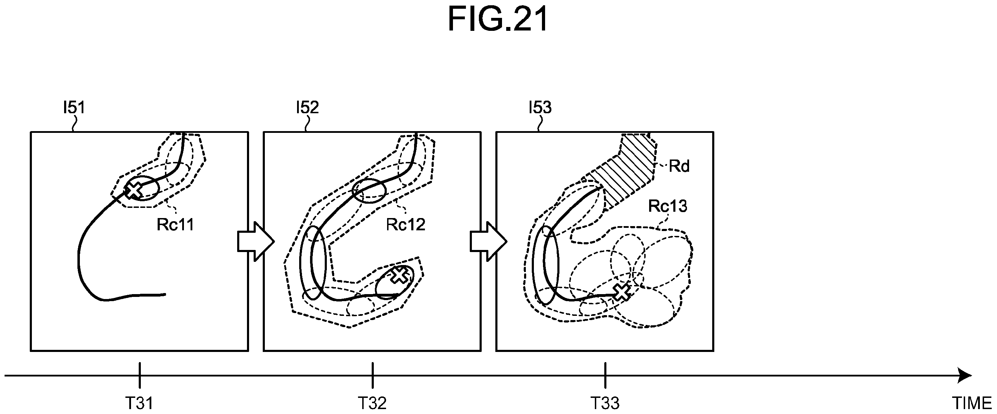

With reference to FIGS. 19 and 20, the dwell mode with consideration of the time elapsed from the determination as the ROIs has been described. Next, a dwell mode with consideration of an ROI area will be described with reference to FIG. 21. FIG. 21 is a diagram illustrating an example of a dwell mode according to the third embodiment; In FIG. 21, an X-ray image I51, an X-ray image I52, and an X-ray image I53 sequentially acquired are illustrated. In addition, the tip end position of a medical device A1 is illustrated with a cross mark in each of the X-ray images I51 to I53.

In FIG. 21, a combined region Rc11 is determined as an ROI at a time T31. In addition, a combined region Rc12 is determined as an ROI at a time T32. In addition, in FIG. 21, the upper limit value is provided for the ROI area. The upper limit value of the ROI area is set, for example, as a percentage of an X-ray image to an area. For example, the upper limit value is set as "30% of the image region". The upper limit value may be adjusted as desired by the user.

Here, for example, in a case where the ROI area reaches the upper limit value at the time T32, but the ROI needs to be extended because the medical device A1 has moved out of the range of the existing ROI, the determination function 110b deletes the ROIs from the earliest one so that the ROI area does not exceed the upper limit value. For example, at a time T33, the determination function 110b can determine, as an ROI, a combined region Rc13 whose ROI area does not exceed the upper limit value by excluding a region Rd corresponding to the early ROI determined before the time T31. That is, the determination function 110b determines the ROI by combining a plurality of regions newly determined out of a plurality of regions determined from sequentially acquired X-ray images so that the ROI area does not exceed the upper limit value.

In a case where no upper limit is set for the ROI area, it is assumed that the ROI is gradually expanded and occupy the majority of the image depending on the situation of the procedure. In this case, the average X-ray conditions are set such that the majority of the image is generally visible, and the visibility of the region of focus is thus not much improved. In contrast, the dwell mode illustrated in FIG. 21 can prevent the ROI from being excessively large and improve the visibility of regions of particular focus, such as the tip end position of the medical device A1 and a new dwell region.

In FIG. 21, the ABC control is also performed based on the degree to which the medical device A1 was dwelling. For example, the calculation function 110c assigns weights to the pixels included in the ROIs as illustrated in FIG. 11. At this time, the calculation function 110c may reduce the weights assigned to pixels at positions where the long time elapsed from the setting of the ROI as illustrated in FIGS. 19 and 20. Alternatively, the calculation function 110c may calculate the parameter related to the ROI based on the weight assigned based on the degree to which the medical device A1 was dwelling without consideration of the time elapsed from the setting of the ROI.

In other words, the dwell mode with consideration of the elapsed time illustrated in FIGS. 19 and 20 and the dwell mode with consideration of the ROI area illustrated in FIG. 21 can be provided as separate modes or combined into a single mode. For example, four dwell modes of a dwell mode with consideration of the time elapsed and the ROI area, a dwell mode with consideration of the time elapsed and without consideration of the ROI area, a dwell mode without consideration of the time elapsed and with consideration of the ROI area, and a dwell mode without consideration of the time elapsed and the ROI area may be provided, and these four dwell modes may be configured to be switchable therebetween according to the user operation or the like.

In addition, the calculation function 110c may retain the information that the region Rd has been included in the ROI after the time T33. Then, for example, in response to an input operation from the user, the control may be switched to include the region Rd in the ROI as appropriate.

As described above, the X-ray diagnosis apparatus 10 can perform the ABC control in various modes. For example, the X-ray diagnosis apparatus 10 can perform the ABC control in the dwell mode with consideration of the time elapsed from the determination as the ROI as described with reference to FIGS. 19 and 20. In addition, the X-ray diagnosis apparatus 10 can perform the ABC control in the dwell mode with consideration of the ROI area as described with reference to FIG. 21. In addition, the X-ray diagnosis apparatus 10 can perform the ABC control in the dwell mode with consideration of the time elapsed from the determination as the ROI and the ROI area. In addition, the X-ray diagnosis apparatus 10 can perform the ABC control in the dwell mode without consideration of the time elapsed from the determination as the ROI and the ROI area. The X-ray diagnosis apparatus 10 can also perform the ABC control in a mode other than the dwell mode, such as the tip end priority mode or the standard mode. The determination function 110b can receive an operation from the user to switch between these modes via the input interface 107. By providing a plurality of modes and switching the modes therebetween as appropriate, the X-ray conditions can be set more appropriately.

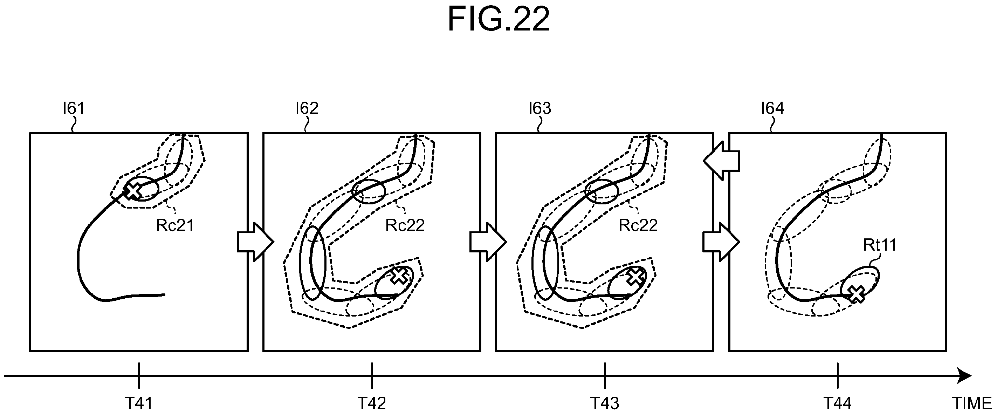

The determination function 110b can switch the modes based on an operation received from the user, or automatic mode switching can be employed under a predetermined condition. An example of automatic mode switching will be described below with reference to FIG. 22. FIG. 22 is a diagram illustrating an example of switching between the modes according to the third embodiment. In FIG. 22, X-ray images I61, I62, I63, and I64 sequentially acquired are illustrated. In addition, the tip end position of a medical device A1 is illustrated with a cross mark in each of the X-ray images I61 to I64. In FIG. 22, a combined region Rc21 is determined as an ROI at a time T41. In addition, a combined region Rc22 is determined as an ROI at a time T42.

In FIG. 22, a combined region Rc22 is determined as an ROI even at a time T43. That is, from the time T42 to the time T43, the tip end of the medical device A1 is not removed from the ROI and the size of the ROI is not changed.

It may be considered, as a case where the size of the ROI is not changed, that the current tip end of the medical device A1 is positioned at a difficult point where it is particularly difficult to advance the medical device A1, or that the current tip end of the medical device A1 is positioned at a site to be treated, but in any cases, it is preferable to switch the current mode to a mode that improves the visibility of the tip end of the medical device A1. For example, the determination function 110b automatically performs switching to a mode that improves the visibility of the tip end of the medical device A1 provided that the amount of change in the size of the ROI is the threshold value or less for a period of time exceeding the threshold value.

An example of a mode that improves the visibility of the tip end of the medical device A1 includes the tip end priority mode. For example, the determination function 110b switches the current mode into the tip end priority mode after a time T44 and determines the ROI to include the current position of the tip end of the medical device A1.

In one example, the determination function 110b determines only a region Rt11 including the tip end of the medical device A1 as the ROI among a plurality of regions illustrated in the X-ray image I64. Alternatively, the determination function 110b may determine an ROI to be a circular region or the like within a predetermined distance from the tip end position of the medical device A1. The calculation function 110c also calculates the statistical values of the pixel values of the pixels included in the ROI as a parameter related to the ROI, and the setting function 110d performs the ABC control based on a parameter related to the ROI. Therefore, the ABC control is performed so that the visibility of the tip end of the medical device A1 is given top priority.

In another example, the determination function 110b determines, as an ROI, a region in which the regions illustrated in the X-ray image I64 are combined. In this case, the ROI includes the region Rt11 that includes the tip end of the medical device A1 and the regions illustrated with the dashed lines in the X-ray image I64. Such an ROI may continue to use ROIs that have been used until the time T43, or may be newly determined along the shape of the medical device A1. The calculation function 110c also assigns a weight to each pixel in the ROI so that the weight of the tip end position of the medical device A1 is the largest. For example, the calculation function 110c assigns a weight to each pixel in the ROI so that the weight is increased as the distance from the tip end of the medical device A1 is small. The calculation function 110c then calculates a parameter related to the ROI based on the assigned weights and pixel values, and the setting function 110d performs the ABC control based on the parameter related to the ROI. Therefore, the ABC control is performed so that the visibility of the tip end and the vicinity of the tip end of the medical device A1 is improved.

An example of a mode that improves the visibility of the tip end of the medical device A1 includes some of the dwell modes. For example, as illustrated in FIG. 11, in a case where a weight of "5" is assigned to the pixel in the region including the tip end, a weight of "3" is assigned to the pixels in the dwell region, and a weight of "1" is assigned to the other pixels in the ROI, the visibility of the tip end is improved while still in the dwell mode.

An example of the mode that improves the visibility of the tip end of the medical device A1 also includes the dwell mode with consideration of the time elapsed, which is described with reference to FIGS. 19 and 20. This is because, although depending on the situation of the procedure, the region with a short time elapsed from the determination as an ROI is often a region near the tip end of the medical device A1. Specifically, while the medical device A1 is simply moving forward without circumstances such as the medical device A1 retreating back to the proximal end side or turning back after passing a branch section and moving forward to another branch destination, the region with a short time elapsed from the determination as the ROI is matched with a region near the tip end of the medical device A1. Thus, for example, when the dwell more without consideration of the time elapsed has been set, the determination function 110b may automatically perform switching to the dwell mode with consideration of the time elapsed provided that the amount of change in the size of the ROI is the threshold value or less for a period of time exceeding the threshold value.

Even after the time T44, the calculation function 110c may retain the ROI or the calculation result of the degree of dwelling until the time T43. Therefore, for example, the current mode can be switched to the tip end priority mode at the time T44, and then switched again to the dwell mode in response to an input operation or the like from the user.

In addition, another case where the ROI is not changed may be when the operation of the medical device A1 has been stopped. In this case, it is rather preferable to reduce the weight assigned to the tip end of the medical device A1 and increase the visibility of the region other than the medical device A1.

In a case where the operation of the medical device A1 has been stopped, the tip end position of the medical device A1 is not moved. On the other hand, In a case where the medical device A1 is positioned at a difficult point or a site to be treated, the tip end position of the medical device A1 is assumed to move finely in the ROI, as illustrated in FIG. 22. Therefore, the determination function 110b may automatically perform switching to the mode that improves the visibility of the tip end of the medical device A1 in a case where the ROI is not changed, and the tip end position of the medical device A1 is moving. For example, the determination function 110b may automatically switch the current mode to the mode that improves the visibility of the tip end of the medical device A1 provided that the amount of change in the size of the ROI is the threshold value or less for a period of time exceeding the threshold value, and the movement amount of the tip end of the medical device A1 exceeds the threshold value.

So far, the switching method between the modes has been described with reference to FIG. 22. However, the series of processes illustrated in FIG. 22 itself can be considered as a type of the dwell mode. That is, the above example described with reference to FIG. 22 can be rephrased as a mode that increases the weights of the pixels near the tip end of the medical device A1 in a case where the ROI is not change. Such a mode is also described as a dwell mode with consideration of the amount of change in an ROI area.

In the case where the dwell mode with consideration of the amount of change in an ROI area is set, the calculation function 110c first calculates a degree to which the medical device A1 was dwelling in an ROI, and assigns weights to a plurality of pixels in the ROI based on the degree. In a case where the amount of change in the size of the ROI is the threshold value or less for a period of time exceeding the threshold value, the calculation function 110c increases the weights of the pixels near the tip end of the medical device A1. For example, in a case where the calculation function 110c assigns a weight of "3" to the pixels in a dwell region and assigns a weight of "1" to the pixels in ROIs other than the dwell region, the weight of the pixels in the region including the tip end of the medical device A1 is changed to "5" provided that the amount of change in the size of the ROI is the threshold value or less for a period of time exceeding the threshold value. In addition, for example, the calculation function 110c assigns the weights to the pixels in the ROI based on the time elapsed from the determination as the ROI provided that the amount of change in the size of the ROI is the threshold value or less for a period of time exceeding the threshold value.

Fourth Embodiment

In addition to the first to third embodiments described above, various other modifications may be implemented.

For example, the above-described embodiment describes an example of identifying a dwell region that requires for the medical device A1 to pass through based on the position of the tip end of the medical device A1 in each of the X-ray images, and assigning the weights to the pixels in the ROIs based on the dwell region. That is, in the above-described embodiment, the example in which the evaluation on the degree to which the medical device A1 was dwelling is performed by using the two-level ranking (whether to correspond to the dwell region or not). However, the evaluation method for the degree to which the medical device A1 was dwelling is not limited thereto.

For example, the calculation function 110c may set three- or more-level ranking indicating the degree to which the medical device A1 was dwelling, identify which rank each pixel in the ROI is classified into based on the position of the tip end of the medical device A1 in each of the X-ray images, and assign a weight to each pixel based on the identified rank. In addition, for example, the calculation function 110c may calculate a value indicating the degree to which the medical device A1 was dwelling with respect to each pixel in the ROI based on the position of the tip end of the medical device A1 in each of the X-ray images, and assign a weight to each pixel based on the calculated value. Such a value can be calculated, for example, based on a movement amount $\Delta X$ described in FIG. 9 or the dwell time described in FIG. 10.

In addition, in the embodiment described above, the examples of various processes such as determining the ROI and assigning a weight based on the position of the tip end of the medical device A1 have been described. However, the embodiments are not limited thereto, and the various processes described above can be executed in the same manner based on positions other than the tip end of the medical device A1. For example, the calculation function 110c can calculates a parameter related to an ROI based on a position of a marker assigned to a position other than the tip end of the medical device A1 and pixel values of a plurality of pixels in the ROI in each of the X-ray images.

In addition, in the embodiment described above, the examples of various processes such as determining the ROI and assigning a weight based on the position of the wire-shaped medical device A1 have been described. However, the embodiments are not limited thereto, and the various processes described above can be executed in the same manner based on positions of a medical device without a wire shape. For example, the calculation function 110c can calculates a parameter related to an ROI based on a position of a marker assigned to a stent and pixel values of a plurality of pixels in the ROI in each of the X-ray images.

In addition, in the above-described embodiment, the example in which the single contiguous region is set as the ROI has been described. However, the embodiments are not limited thereto, and a plurality of separated regions may be set as ROIs.

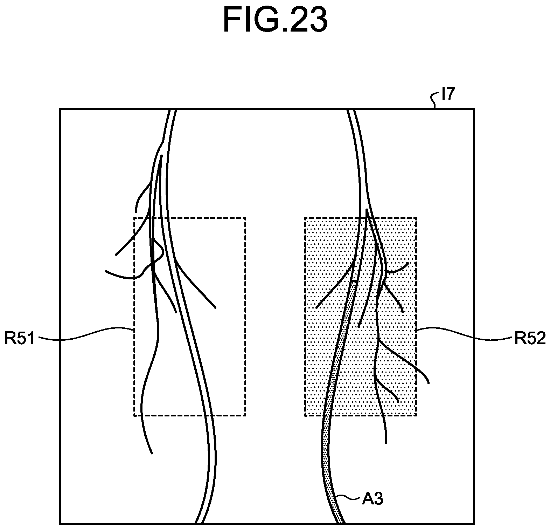

For example, as illustrated in FIG. 23, a user may focus on a plurality of unconnected vascular regions on an X-ray image 17. In this case, the determination function 110b determines a region R51 and a region R52 set for individual vessel regions as ROIs. In this case, the calculation function 110c may vary weights between regions. For example, in the case illustrated in FIG. 23, the calculation function 110c may set larger weights to pixels in the region R52 than to pixels in the region R51, because a medical device A3 is inserted within the vascular region where the R52 is set.

In addition, in the embodiment described above, a C-arm type device in which the X-ray tube 102 and the X-ray collimator 103 are held by the C-arm 105 is illustrated as an example of the X-ray diagnosis apparatus 10. However, the embodiments are not limited thereto. For example, the X-ray tube 102 may be held by a different mechanism than the C-arm 105, such as an arm that can travel on rails in the ceiling. Any modifications can be applied to the specific configuration of the X-ray diagnosis apparatus 10 as long as it is possible to acquire the X-ray images from the subject over time.

In addition, in the embodiment described above, the example in which various functions such as the determination function 110b, the calculation function 110c, and the setting function 110d are performed in the processing circuitry 110 included in the X-ray diagnosis apparatus 10 has been described, but the embodiments are not limited thereto. That is, the X-ray condition setting method described above may be implemented in a device different from the X-ray diagnosis apparatus 10. For example, the X-ray conditions may be set by processing circuitry included in an information processing device connected to the X-ray diagnosis apparatus 10 via a network to execute functions equivalent to the determination function 110b, the calculation function 110c, and the setting function 110d.

For example, the X-ray diagnosis apparatus 10 acquires a plurality of X-ray images over time from a subject P into which a medical device is inserted, and sequentially transmits the acquired X-ray images to the information processing device via the network. The information processing device determines a region including the medical device in the received X-ray images in the same manner performed by the determination function 110b. In addition, similar to the calculation function 110c, the information processing device calculates a parameter related to the determined region based on the position of the medical device in each of the X-ray images and pixel values of a plurality of pixels included in the region. In addition, similar to the setting function 110d, the information processing device sets X-ray conditions based on the parameter. The information processing device then transmits the set X-ray conditions to the X-ray diagnosis apparatus 10 via the network. Therefore, the X-ray diagnosis apparatus 10 can continue to acquire X-ray images under the appropriate X-ray conditions set by the information processing device.

The network in the above-described embodiments may include a local network closed within the hospital, or may be a network via the Internet. For example, the information processing device that executes the X-ray condition setting method described above may be installed in the same facility as the X-ray diagnosis apparatus 10, or in a different facility.

The term "processor" used in the above description means circuitry such as CPU, graphics processing unit (GPU), ASIC, programmable logic device (for example, simple programmable logic device (SPLD), complex programmable logic device (CPLD), and field programmable gate array (FPGA)), for example. In a case where the processor is, for example, CPU, the processor reads out and executes the computer programs stored in a memory circuit to execute the functions. On the other hand, in a case where the processor is, for example, ASIC, instead of storing the computer program in the memory circuit, the functions are incorporated directly as a logic circuit within the circuitry of the processor. Each processor of the embodiments is not limited to the configuration of a single circuit provided for each processor, but may also employ the configuration of a single processor formed of a plurality of independent circuits in combination to implement the functions thereof. Furthermore, a plurality of components in each figure may be integrated into a single processor to achieve the functions.

Each of the components of each of the devices according to the above-described embodiments is a functional concept, and does not necessarily has the physical configuration as illustrated in the figures. That is, the specific form of distribution and integration of each of the devices is not limited to those illustrated in the figures, and all or some of the devices can be functionally or physically distributed and integrated in any units according to various loads and usage conditions. Furthermore, all or some parts of the processing functions performed by the devices can be implemented by CPU and a computer program analyzed and executed by the CPU, or by hardware using wired logic.

In addition, the X-ray condition setting method described in the embodiments described above can be implemented by executing a prepared medical information processing program on a computer such as a personal computer or workstation. This medical information processing program can be distributed via the Internet or other networks. The medical information processing program can also be recorded on a non-transitory recording medium readable by a computer, such as hard disk, flexible disk (FD), CD-ROM, MO, DVD, or the like, and executed by the computer reading out the computer program from the recording medium.

According to at least one of the embodiments described above, the X-ray conditions can be set appropriately.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. An X-ray diagnosis apparatus (10) comprising:

an acquisition unit (110a) configured to acquire a plurality of X-ray images sequentially from a subject (P) with a medical device (A1, A3) inserted into a body of the subject (P);
a determination unit (110b) configured to determine a region including the medical device (A1, A3) in the X-ray images;
a calculation unit (110c) configured to calculate a degree to which the medical device (A1, A3) is dwelling in the region based on the X-ray images, assign weights to a plurality of pixels included in the region based on the degree, and calculate a parameter related to the region based on the weights and pixel values of the pixels; and
a setting unit (110d) configured to set an X-ray condition based on the parameter.

2. The X-ray diagnosis apparatus (10) according to claim 1, wherein the calculation unit (110c) is configured to determine a movement amount of the medical device (A1, A3) between the X-ray images based on a position of the medical device (A1, A3) in each of the X-ray images, and evaluate the degree based on the movement amount.

3. The X-ray diagnosis apparatus (10) according to claim 1, wherein the calculation unit (110c) is configured to determine a length of time when the medical device (A1, A3) is dwelling based on a position of the medical device (A1, A3) in each of the plurality of X-ray images, and evaluate the degree based on the length of time.

4. The X-ray diagnosis apparatus (10) according to claim 1, wherein the calculation unit (110c) is configured to calculate a parameter related to the region based on a position of a tip end of the medical device (A1, A3) in each of the X-ray images and the pixel values.

5. The X-ray diagnosis apparatus (10) according to claim 1, wherein the calculation unit (110c) is configured to calculate a parameter related to the region based on a position of a marker assigned to the medical device (A1, A3) in each of the X-ray images and the pixel values.

6. The X-ray diagnosis apparatus (10) according to claim 1, wherein the calculation unit (110c) is configured to assign the weights based on the degree and a time elapsed from determination as the region, and increase the weights of pixels near a tip end of the medical device (A1, A3) provided that an amount of change in a size of the region is a threshold value or less for a period of time exceeding the threshold value.

7. The X-ray diagnosis apparatus (10) according to claim 1, wherein the determination unit (110b) is configured to change a shape between a first region and a second region in steps when a dwell mode and a standard mode are switched, wherein:

in the dwell mode, the first region including the medical device (A1, A3) in the X-ray images is determined as the region, the degree to which the medical device (A1, A3) is dwelling in the first region is calculate, the weights is assigned to a plurality of pixels in the first region based on the degree, the parameter is calculated based on the weights and pixel values of the pixels, and the X-ray condition is set based on the parameter; and
in the standard mode, a parameter related to the second region, which is predetermined, is calculated based on pixel values of a plurality of pixels included in the second region, and the X-ray condition is set based on the parameter.

8. The X-ray diagnosis apparatus (10) according to claim 1, wherein the calculation unit (110c) is configured to assign a first weight to pixels in a dwell region where the degree is high, assign a second weight smaller than the first weight to pixels in the region except for the dwell region, and replace weights assigned to the pixels in the dwell region by the second weight in a case where a time elapsed from determination as the region exceeds a threshold value.

9. The X-ray diagnosis apparatus (10) according to claim 1, wherein the determination unit (110b) is configured to determine the region by combining a plurality of regions newly determined out of a plurality of regions determined from sequentially acquired X-ray images so that an area of the region does not exceed an upper limit value.

10. The X-ray diagnosis apparatus (10) according to claim 1, wherein the determination unit (110b) is configured to receive an input operation from a user to perform mode switching between a plurality of modes including a dwell mode including assigning the weights to a plurality of pixels included in the region based on the degree.

11. The X-ray diagnosis apparatus (10) according to claim 10, wherein the determination unit (110b) is configured to perform switching to a mode that improves a visibility of a tip end of the medical device (A1, A3) provided that an amount of change in a size of the region is a threshold value or less for a period of time exceeding the threshold value.

12. The X-ray diagnosis apparatus (10) according to claim 1, wherein

the determination unit (110b) is configured to determine a second region by processing a first region, and
the calculation unit (110c) is configured to calculate the parameter based on a position of the medical device (A1, A3) in each of the X-ray images and pixel values of a plurality of pixels included in the second region.

13. The X-ray diagnosis apparatus (10) according to claim 1, further comprising an output unit (110e) configured to display the region on a display unit.

14. The X-ray diagnosis apparatus (10) according to claim 13, wherein the output unit sequentially displays newly acquired X-ray images on the display unit (108) and displays the region on the X-ray images for regular time interval in a superimposing manner.

15. An X-ray condition setting method comprising:

determining a region including a medical device (A1, A3) in a plurality of X-ray images sequentially acquired from a subject (P) with the medical device (A1, A3) inserted into a body of the subject (P);
calculating a degree to which the medical device (A1, A3) is dwelling in the region based on the X-ray images, assigning weights to a plurality of pixels included in the region based on the degree, and calculating a parameter related to the region based on the weights and pixel values of the pixels; and
setting an X-ray condition based on the parameter.

# FIG.1

10

106 — X-RAY DETECTOR

105

P

104

103 — X-RAY COLLIMATOR

102 — X-RAY TUBE

101 — X-RAY HIGH VOLTAGE DEVICE

INPUT INTERFACE — 107

DISPLAY — 108

MEMORY — 109

PROCESSING CIRCUITRY — 110

ACQUISITION FUNCTION — 110a

DETERMINATION FUNCTION — 110b

CALCULATION FUNCTION — 110c

SETTING FUNCTION — 110d

OUTPUT FUNCTION — 110e

# FIG.2

# FIG.3

# FIG.4

```
        START
          │
          ▼
┌─────────────────────┐
│ INITIATE X-RAY IMAGE │──── S101
│    ACQUISITION       │
└─────────────────────┘
          │
          ▼
┌─────────────────────┐
│    DETERMINE ROI     │──── S102
└─────────────────────┘
          │
          ▼
┌─────────────────────┐
│   SET WEIGHT FOR     │──── S103
│     EACH PIXEL       │
└─────────────────────┘
          │
          ▼
┌─────────────────────┐
│     CALCULATE        │──── S104
│     PARAMETER        │
└─────────────────────┘
          │
          ▼
┌─────────────────────┐
│     SET X-RAY        │──── S105
│    CONDITIONS        │
└─────────────────────┘
          │
          ▼
        ◇ IS ACQUISITION ◇ ──── S106
   NO   ◇  COMPLETED?    ◇
          │ YES
          ▼
         END
```

# FIG.5

# FIG.6

R23

TIP END POSITION IN I22'

TIP END POSITION IN I23'

A1

# FIG.7

R22

R21

R23

R24

R26

R25

# FIG.8

I2

TRAJECTORY OF
TIP END

A2

DWELL REGION

# FIG.9

X-RAY IMAGE ACQUIRED
n FRAMES BEFORE X-RAY IMAGE I25

X-RAY IMAGE I25

DIFFER-
ENTIAL

MOVEMENT
AMOUNT $\Delta X$

# FIG.10

X-RAY IMAGE I26 OF FRAME f

# FIG.11

| 0 | 0 | 0 | 0 | 0 | 1 | 1 | 0 |
|---|---|---|---|---|---|---|---|
| 0 | 0 | 0 | 1 | 1 | 1 | 0 | 0 |
| 0 | 0 | 1 | 1 | 0 | 0 | 0 | 0 |
| 0 | 0 | 1 | 0 | 0 | 0 | 0 | 0 |
| 0 | 0 | 3 | 1 | 0 | 0 | 0 | 0 |
| 0 | 0 | 3 | 1 | 1 | 5 | 0 | 0 |
| 0 | 0 | 0 | 0 | 0 | 0 | 0 | 0 |
| 0 | 0 | 0 | 0 | 0 | 0 | 0 | 0 |

DWELL REGION   TIP END

# FIG.12

ROI

DWELL REGION

# FIG.13

ROI

DWELL REGION

# FIG.14

| MENU |
|---|
| ABCROI<br>ON/OFF |
| DEVICE DISPLAY |
| TIP END POSITION DISPLAY |
| STANDARD ROI |
| RESET |

# FIG.15

STANDARD ROI ←——————————————————→ MEDICAL DEVICE A1

EP 4 578 400 A1

# FIG.16

124'

# FIG.17

# FIG.18

# FIG.19

EP 4 578 400 A1

# FIG.20

EP 4 578 400 A1

# FIG.21

# FIG.22

# FIG.23

# EUROPEAN SEARCH REPORT

**Application Number**

EP 24 22 3232

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 2016 007435 A (TOSHIBA CORP; TOSHIBA MEDICAL SYS CORP) 18 January 2016 (2016-01-18) * paragraph [0064] * ----- | 1-15 | INV. A61B6/12 A61B6/46 |
| A | JP 2015 211914 A (TOSHIBA CORP; TOSHIBA MEDICAL SYS CORP) 26 November 2015 (2015-11-26) * paragraph [0153] * ----- | 1-15 | |

**TECHNICAL FIELDS SEARCHED      (IPC)**

A61B

The present search report has been drawn up for all claims

| Place of search | Date of completion of the search | Examiner |
|---|---|---|
| Munich | 3 April 2025 | Anscombe, Marcel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................

& : member of the same patent family, corresponding
document

## ANNEX TO THE EUROPEAN SEARCH REPORT
## ON EUROPEAN PATENT APPLICATION NO.

EP 24 22 3232

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2025

| Patent document cited in search report | Publication date | Patent family member(s) | Publication date |
|---|---|---|---|
| JP 2016007435 A | 18-01-2016 | JP 6373654 B2 | 15-08-2018 |
| | | JP 2016007435 A | 18-01-2016 |
| JP 2015211914 A | 26-11-2015 | JP 6117299 B2 | 19-04-2017 |
| | | JP 2015211914 A | 26-11-2015 |

For more details about this annex : see Official Journal of the European Patent Office, No. 12/82